# EUROPEAN PATENT SPECIFICATION

(11) **EP 4 182 480 B1**
(45) Date of publication and mention of the grant of the patent: **07.05.2025**
(21) Application number: 21739731.4
(22) Date of filing: 16.07.2021
(51) Int. Cl.: C12Q 1/6886

(54) **PROGNOSTIC BIOMARKERS FOR CANCER**
PROGNOSTISCHE BIOMARKER FÜR KREBS
BIOMARQUEURS DE PRONOSTIC POUR LE CANCER

(30) Priority: 17.07.2020 EP 20186398
(43) Date of publication of application: 24.05.2023
(73) Proprietor: European Molecular Biology Laboratory, 69117 Heidelberg (DE); Universität Bern, 3012 Bern (CH)
(72) Inventor: LINDER, Bastian, 69117 Heidelberg (DE); STEINMETZ, Lars, 69117 Heidelberg (DE); LEIDEL, Sebastian, 3012 Bern (CH)
(74) Representative: Kuttenkeuler, David
(86) International application number: PCT/EP2021/069952
(87) International publication number: WO 2022/013420

(56) References cited:
- WO-A1-2018/075947
- WO-A1-2019/178217
- WO-A1-2019/183417
- WO-A1-2019/197024
- WO-A1-2021/116477
- BARBIERI ISAIA ET AL: "Role of RNA modifications in cancer", NATURE REVIEWS CANCER, NATURE PUB. GROUP, LONDON, vol. 20, no. 6, 16 April 2020 (2020-04-16), pages 303 - 322, XP037149667, ISSN: 1474-175X, [retrieved on 20200416], DOI: 10.1038/S41568-020-0253-2
- LEONARDI ANDREA ET AL: "Epitranscriptomic systems regulate the translation of reactive oxygen species detoxifying and disease linked selenoproteins", FREE RADICAL BIOLOGY & MEDICINE, ELSEVIER INC, US, vol. 143, 30 August 2019 (2019-08-30), pages 573 - 593, XP085901745, ISSN: 0891-5849, [retrieved on 20190830], DOI: 10.1016/J.FREERADBIOMED.2019.08.030
- LI YUE ET AL: "Expression of Demethylase Genes,FTOandALKBH1, Is Associated with Prognosis of Gastric Cancer", DIGESTIVE DISEASES AND SCIENCES, SPRINGER NEW YORK LLC, US, vol. 64, no. 6, 14 January 2019 (2019-01-14), pages 1503 - 1513, XP036784551, ISSN: 0163-2116, [retrieved on 20190114], DOI: 10.1007/S10620-018-5452-2
- FABBRI LUCILLA ET AL: "The plasticity of mRNA translation during cancer progression and therapy resistance", NATURE REVIEWS CANCER, NATURE PUB. GROUP, LONDON, vol. 21, no. 9, 2 August 2021 (2021-08-02), pages 558 - 577, XP037545744, ISSN: 1474-175X, [retrieved on 20210802], DOI: 10.1038/S41568-021-00380-Y
- ESTEVE-PUIG ROSAURA ET AL: "Writers, readers and erasers of RNA modifications in cancer", CANCER LETTERS, NEW YORK, NY, US, vol. 474, 25 January 2020 (2020-01-25), pages 127 - 137, XP086030018, ISSN: 0304-3835, [retrieved on 20200125], DOI: 10.1016/J.CANLET.2020.01.021
- JUNHONG CHOI ET AL: "N6-methyladenosine in mRNA disrupts tRNA selection and translation-elongation dynamics", NATURE STRUCTURAL & MOLECULAR BIOLOGY, vol. 23, no. 2, 11 January 2016 (2016-01-11), New York, pages 110 - 115, XP055756345, ISSN: 1545-9993, DOI: 10.1038/nsmb.3148
- NICKY JONKHOUT ET AL: "The RNA modification landscape in human disease", RNA, vol. 23, no. 12, 16 December 2017 (2017-12-16), US, pages 1754 - 1769, XP055516059, ISSN: 1355-8382, DOI: 10.1261/rna.063503.117
- PAN TAO: "Modifications and functional genomics of human transfer RNA", CELL RESEARCH, SPRINGER SINGAPORE, SINGAPORE, vol. 28, no. 4, 20 February 2018 (2018-02-20), pages 395 - 404, XP036800629, ISSN: 1001-0602, [retrieved on 20180220], DOI: 10.1038/S41422-018-0013-Y
- R. JORDAN ONTIVEROS ET AL: "Coordination of mRNA and tRNA methylations by TRMT10A", PROCEEDINGS OF THE NATIONAL ACADEMY OF SCIENCES, vol. 117, no. 14, 25 March 2020 (2020-03-25), pages 7782 - 7791, XP055756672, ISSN: 0027-8424, DOI: 10.1073/pnas.1913448117
- RAMOS JILLIAN ET AL: "The emerging impact of tRNA modifications in the brain and nervous system", BIOCHIMICA ET BIOPHYSICA ACTA. GENE REGULATORY MECHANISMS, vol. 1862, no. 3, pages 412 - 428, XP085616575, ISSN: 1874-9399, DOI: 10.1016/J.BBAGRM.2018.11.007
- DEDON PETER C ET AL: "Dysfunctional tRNA reprogramming and codon-biased translation in cancer", TRENDS IN MOLECULAR MEDICINE, ELSEVIER CURRENT TRENDS, GB, vol. 28, no. 11, 11 October 2022 (2022-10-11), pages 964 - 978, XP087218279, ISSN: 1471-4914, [retrieved on 20221011], DOI: 10.1016/J.MOLMED.2022.09.007
- "Affymetrix Human Genome U133 Plus 2.0 Array [CDF: HGU133Plus2_Hs_ENSG v12.1.0]", XP002668741, Retrieved from the Internet <URL:http://www.ncbi.nlm.gov/geo/quera/acc.cgi?acc=GPL9987> [retrieved on 20100419]
- DUECHLER MARKUS ET AL: "Nucleoside modifications in the regulation of gene expression: focus on tRNA", CMLS CELLULAR AND MOLECULAR LIFE SCIENCES, BIRKHAUSER VERLAG, HEIDELBERG, DE, vol. 73, no. 16, 19 April 2016 (2016-04-19), pages 3075 - 3095, XP036009158, ISSN: 1420-682X, [retrieved on 20160419], DOI: 10.1007/S00018-016-2217-Y
- KAPUR MRIDU ET AL: "mRNA Translation Gone Awry: Translation Fidelity and Neurological Disease", TRENDS IN GENETICS, ELSEVIER SCIENCE PUBLISHERS B.V. AMSTERDAM, NL, vol. 34, no. 3, 16 January 2018 (2018-01-16), pages 218 - 231, XP085353605, ISSN: 0168-9525, DOI: 10.1016/J.TIG.2017.12.007

## Description

### FIELD OF THE INVENTION

The present invention pertains to a new *in-vitro* method for the prognosis, diagnosis, stratification and/or monitoring of a therapy, of a disorder in a subject. The *in-vitro* method is based on the determination of the level of at least one first biomarker indicative and/or selective for the status and/or activity of the pathway regulating N6-methyladenosine (m6A) mRNA modification, and the determination of the level, mutation status, and/or activity of at least one second biomarker indicative and/or selective for the status and/or activity of the pathway regulating 5-methoxycarbonylmethyl-2-thiouridine (mcm5s2U)-tRNA modification as combined biomarkers. The new biomarkers of the invention allow to estimate a patient's prognosis, and to monitor therapy success of patients, in particular cancer patients, such as breast cancer patients. The biomarkers of the invention furthermore allow diagnosing and even stratifying of a disorder in a patient, in particular cancer, such as breast cancer.

### DESCRIPTION

Breast cancer is the most frequent cancer among women, and the cause of the greatest number of cancer-related deaths among women. About 12% of U.S. women will develop invasive breast cancer over the course of her lifetime. Currently, breast cancer is impacting around 2.1 million women each year. In 2018, it is estimated that 627,000 women died from breast cancer, which is approximately 15% of all cancer deaths among women. Breast cancer may generally be divided into several main stages: early, locally advanced, locally recurrent and metastatic. The 5-year survival prognosis for early stage breast cancer is generally above 60%, and this number drops to between 40 and 60% for advanced breast cancer.

Current therapeutic options for treatment of breast cancer include surgery (e.g. resection, autologous bone marrow transplantation), radiation therapy, chemotherapy (e.g. anthracyclines, such as doxorubicin; alkylating agents, such as cyclophosphamide and mitomycin C; taxanes, such as paclitaxel and docetaxel; antimetabolites, such as capecitabine; microtubule inhibitors, such as the vinca alkaloid navelbine; endocrine therapy (e.g. antiestrogens, such as tamoxifen; progestins, such as medroxyprogesterone acetate and megastrol acetate; or aromatase inhibitors, such as aminoglutethamide and letrozole) and biologics (e.g. cytokines, immunotherapeutics, such as monoclonal antibodies).

Diagnostic options for breast cancer include microscopic analysis of a patient's sample - or biopsy - obtained from the affected area of the breast of the patient. The two most commonly used diagnostic approaches are physical examination of the breasts by a medical practitioner and mammography. When these examinations are inconclusive, usually a patient sample of the fluid in the lump is sent for microscopic analysis to help establish the diagnosis. Other options for biopsy include computer tomography (CT), magnet resonance imaging (MRI), an ultrasound examination and a core biopsy or vacuum-assisted breast biopsy, or an excisional biopsy, in which the entire breast lump is removed. In order to improve cancer outcomes and survival, early detection of cancer, such as breast cancer, is critical.

During the past decades, many factors have been validated to have predictive value for the prognosis of cancer patients. For example, in case of breast cancer patients, hormone receptor (estrogen/progesterone receptor, ER/PR) positive and human epidermal growth factor receptor 2 (HER2) negative phenotypes are well documented to be associated with a favorable prognostic outcome compared to women with ER/PR negative and/or HER2 positive disease.

WO2006036726A1 discloses a method for evaluating the prognosis of a breast cancer patient, wherein said method comprises the detection of at least five biomarkers (SLPI, src, PSMB9, p21ras, and E2F1).

US20110097732A1 discloses a method for determining the prediction for survival of an individual having breast cancer by measuring the amount of Wrap53 in the nucleus of cells in samples derived from breast tissue, and/or measuring the amount of Wrap53 in the cytoplasm of cells in the samples derived from breast tissue, wherein both the nuclear levels, the cytoplasmic levels and/or the ratio between the nuclear and cytoplasmic levels of Wrap53 may be used alone or in combination in breast cancer prognosis. However, the disadvantage of this method is that tissue needs to be obtained from an open surgery, or prior to surgery from a fine needle biopsy or a core needle biopsy.

So far, no approach for a minimal invasive but yet specific and sensitive test system for the prognosis and/or monitoring of diseases, such as cancer diseases, has been developed, which pertains to analyzing the epitranscriptomic layer of gene regulation.

Despite its widespread occurrence, the functions of the "epitranscriptomic" layer of gene regulation is not yet fully understood. The main function of mRNA is to template protein synthesis during translation on the ribosome. In this process, the genetic information is decoded by tRNAs via base-pairing of their anticodon loop and the mRNA codon. More than 200 different chemically modified nucleotides have been identified in a variety of RNA species including mRNA, tRNA, rRNA and ncRNA. This "epitranscriptome" alters the expression and/or function of the modified RNAs and thus represents a layer of gene regulation. As an example, the anticodon loop of tRNAs is a "hotspot" for RNA modifications and these have been implicated in gene expression control during stress.

With a modification rate of up to 0.5% of all adenosines, the modified mRNA nucleotide N6-methyladenosine (m6A) is the most abundant modification in mRNA. m6A is co-transcriptionally deposited on nascent RNAs during their transcription by RNA polymerase II by the m6A methyltransferase complex (m6A-MTC), which consists of METTL3, METTL14, WTAP, VIRMA, RBM15 ZC3H13. Thereby, m6A imprints mRNAs with splicing information that is read by tRNAs.

The m6A consensus sequence is referred to as "DRACH motif" (D = A, G, or U; R = G or A; H = A, C or U). The DRACH motif appears once every ~57 nucleotides in mRNA, and its transcription generates a potential methylation site (DR**A**CH). Modification with m6A results in a decrease in mRNA half-life, i.e. an increased turnover of the modified mRNAs in cells. The m6A "reader" proteins YTHDF2 and YTHDF3 have been implicated in this process, but how degradation is triggered is unknown. Nevertheless, m6A-mediated decay was shown to be important to remodel cellular transcriptomes during cellular transitions like differentiation or stress adaptation. Accordingly, m6A is implicated in a variety of health conditions in which cellular homeostasis is perturbed, e.g. in cancer, viral infections or cardiac failure.

A major function of m6A-mediated mRNA decay is to remove pluripotency genes to facilitate the differentiation of stem cells [1,2]. m6A was also shown to be involved in various cancers, including glioblastoma, acute myeloid leukemia, bladder cancer and hepatocellular carcinoma. Therefore, drugging the epitranscriptome by targeting m6A is a promising therapy for diseases, such as cancer. Drugs that target m6A biogenesis factors are currently being developed and first lead molecules exist, but it is unclear which patients will benefit most from these drugs. Also, some of the initial studies reported contradictory results on whether m6A acts as either an oncogene or a tumor suppressor, underlining the need to establish methods that predict treatment outcome and enable stratification of patients into groups that benefit from compounds activating m6A and/or patients that benefit from compounds inhibiting m6A.

WO2019197024A1 is an application concerning subject matter generally related to the epitranscriptomic regulation of ribonucleic acid (RNA) methylation through small-molecule activators. It discloses an expression analysis of the activity level of METTL3/METTL14/ WTAP which affect m6A levels, and discloses the medical use of compounds affecting the activity of METTL3/METTL14/ WTAP to increase m6A levels.

WO2019183417A1 is an application that concerns methods and compositions for reducing drug resistance. It discloses a determination of m6A levels and provides compounds affecting m6A levels.

WO2019178217A1 relates to methods and compositions for the treatment of cancers in a patient with a particular biomarker profile. Amongst others, it discloses an expression signature of m6A regulators for determining prognosis in human cancers.

A major step in many aspects of research related to diseases such as cancer is the identification of specific and sensitive biomarkers suitable for the development of effective and improved prognostic, diagnostic, and therapeutic modalities. In view of the above described background art, the objective of the present invention is to provide novel markers for use in the prognosis, diagnosis, and/or monitoring of a disease, condition or disorder in a subject, and for use in the development of novel therapeutics. Due to the continuing need for quick, but sensitive and specific cancer prognostic and diagnostic markers, the present invention seeks to provide a novel approach for a simple and minimal invasive but specific and sensitive test system for the prognosis, diagnosis, and/or monitoring of a disease, condition or disorder in a subject, such as cancer. Furthermore, the present invention seeks to provide diagnostic strategies that allow to directly monitor the ongoing treatment success of a therapy in the clinic, such as a cancer therapy, and to evaluate the prognosis for a cancer patient receiving the therapy.

The above problem is solved by providing novel markers for use in the prognosis, diagnosis, and/or monitoring of a disease, condition or disorder in a subject, and for use in the development of novel therapeutics, which are based on analyzing the interdependency of the m6A system and an tRNA anticodon modification, namely 5-methoxycarbonylmethyl-2-thiouridine (mcm5s2U).

### BRIEF DESCRIPTION OF THE INVENTION

Generally, and by way of brief description, the main aspects of the present invention can be described as follows:

In a **first aspect,** the invention pertains to the use of a biomarker consisting of a N6-methyladenosine (m6A)-mRNA modification and a 5-methoxycarbonylmethyl-2-thiouridine (mem5s2U)-tRNA anticodon modification in an *in-vitro* method for the prognosis, diagnosis, stratification and/or monitoring of a disease, condition or disorder in a subject.

In a **second aspect,** the invention pertains to the use of a kit in an *in-vitro* method for the prognosis, diagnosis, and/or monitoring of a disease, condition or disorder in a subject as defined in the claims, the kit comprising means for quantifying the level, mutation status, and/or activity of the at least one m6A-mRNA modification biomarker indicative and/or selective for the status and/or activity of the pathway regulating m6A-mRNA modification in a cell, and/or for quantifying the level, mutation status, and/or activity of the at least one mcm5s2U-tRNA anticodon modification biomarker indicative and/or selective for the status and/or activity of the pathway regulating mcm5s2U-tRNA anticodon modification in a cell.

### DETAILED DESCRIPTION OF THE INVENTION

In the following, the elements of the invention will be described. These elements are listed with specific embodiments, however, it should be understood that they may be combined in any manner and in any number to create additional embodiments. The variously described examples and preferred embodiments should not be construed to limit the present invention to only the explicitly described embodiments. This description should be understood to support and encompass embodiments which combine two or more of the explicitly described embodiments or which combine the one or more of the explicitly described embodiments with any number of the disclosed and/or preferred elements. Furthermore, any permutations and combinations of all described elements in this disclosure should be considered disclosed by the description of the present disclosure unless the context indicates otherwise.

In a **first aspect,** the invention pertains to the use of a biomarker consisting of a N6-methyladenosine (m6A)-mRNA modification and a 5-methoxycarbonylmethyl-2-thiouridine (mem5s2U)-tRNA anticodon modification in an *in-vitro* method for the prognosis, diagnosis, stratification and/or monitoring of a disease, condition or disorder in a subject.

The invention pertains to an *in-vitro* method for the prognosis, diagnosis, and/or monitoring of a disease, condition or disorder in a subject, comprising the steps of
**(a)** Providing a biological sample of said subject,
**(b)** Determining the level, mutation status, and/or activity of at least one first biomarker indicative and/or selective for the status and/or activity of the pathway regulating N6-methyladenosine (m6A) mRNA modification in a cell in said biological sample,
**(c)** Determining the level, mutation status, and/or activity of at least one second biomarker indicative and/or selective for the status and/or activity of the pathway regulating 5-methoxycarbonylmethyl-2-thiouridine (mem5s2U)-tRNA modification in a cell in said biological sample, and
**(d)** Comparing the determined level, mutation status, and/or activity of said first and said second biomarker in steps **(b)** and **(c)** with a reference sample or a reference value,
wherein a differential level, mutation status, and/or activity of said first and/or second biomarker in the biological sample from said subject compared to said reference sample or reference value is indicative for a reduced time of progression free survival (PFS) and/or overall survival (OS) of a patient suffering from a disease, condition or disorder, or wherein a differential level, mutation status, and/or activity of said first and/or second biomarker in the biological sample from said subject compared to said reference sample or reference value is indicative for the presence of said disease, condition or disorder in said subject.

The term "prognosis" refers to a forecast as to the probable outcome of the disease as well as the prospect of recovery from the disease as indicated by the nature and symptoms of the case. Accordingly, a negative or poor prognosis is defined by a lower post-treatment survival term or survival rate. Conversely, a positive or good prognosis is defined by an elevated post-treatment survival term or survival rate. Usually prognosis is provided as the time of progression free survival (PFS) or overall survival (OS).

Progression-free survival (PFS), as used in the context of this invention, shall be defined as the length of time during and after the treatment of a disease, such as cancer, that a patient lives with the disease but the disease does not get worse, i.e. survival without progression of the disease. PFS shall further be defined as the time from random assignment, e.g. in a clinical trial, to disease progression or death from any cause. In a clinical trial, measuring the PFS is one way to see how well a new treatment works. The term "overall survival" (OS) as used in the context of this invention shall be defined as the duration of time from the date of diagnosis or commencement of a treatment of a particular disease that a patient is still alive. Thus, in a clinical trial the measure of overall survival would compare the number of patients who had died and the number who had not died.

A "diagnosis" or the term "diagnostic" in the context of the present invention means identifying the presence or nature of a pathologic condition. Diagnostic methods differ in their sensitivity and specificity. The "sensitivity" of a diagnostic assay is the percentage of diseased individuals who test positive (percent of "true positives"). Diseased individuals not detected by the assay are "false negatives." Subjects who are not diseased and who test negative in the assay, are termed "true negatives." The "specificity" of a diagnostic assay is 1 minus the false positive rate, where the "false positive" rate is defined as the proportion of those without the disease who test positive. While a particular diagnostic method may not provide a definitive diagnosis of a condition, it suffices if the method provides a positive indication that aids in diagnosis.

The term "monitoring of a disease, condition or disorder" shall mean for the purpose of the present invention to observe disease progression in a patient who receives a therapy. In other words, the patient during the therapy is regularly monitored for the effect of the applied therapy, which allows the medical practitioner to estimate at an early stage during the therapy whether the prescribed treatment is effective or not, and, therefore, to adjust the treatment regime accordingly.

According to the present invention, any disease, condition or disorder can be prognosed, diagnosed, and/or monitored, using the *in-vitro* methods of this invention. In a preferred embodiment, said disease, condition or disorder to be prognosed, diagnosed, and/or monitored is cancer, a viral disease, a cardiac disease, a neurodegenerative disease, a disease associated with oxidative stress, and/or other disease that shows a differential level, mutation status, and/or activity of the pathway regulating N6-methyladenosine (m6A) mRNA modification in a cell, and/or that show a differential level, mutation status, and/or activity of the pathway regulating 5-methoxycarbonylmethyl-2-thiouridine (mcm5s2U)-tRNA modification in a cell.

In a further preferred embodiment, said cancer to be prognosed, diagnosed, and/or monitored is selected from breast cancer, melanoma, glioblastoma, endometrial cancer, Acute Myeloid Leukemia (AML), lung cancer, bladder cancer, hepatocellular cancer, medulloblastoma, kidney cancer, liver cancer, head and neck cancer, prostate cancer, pancreatic cancer, stomach cancer, colon cancer, thyroid cancer, esophageal cancer, brain cancer, colorectal cancer, gastric cancer, cervical cancer, ovarian cancer, cancer of the urinary tract, renal cancer, carcinoma, and a tumor that shows a differential level, mutation status, and/or activity of the pathway regulating m6A-mRNA modification in a cell, and/or regulating mcm5s2U-tRNA modification in a cell, preferably wherein said cancer is breast cancer, melanoma, glioblastoma, endometrial cancer, Acute Myeloid Leukemia (AML), lung cancer, bladder cancer, hepatocellular cancer, medulloblastoma, or a tumor that shows a differential level, mutation status, and/or activity of the pathway regulating m6A-mRNA modification in a cell, and/or regulating mcm5s2U-tRNA modification in a cell. Most preferably, said cancer is breast cancer.

The term "cancer" and "cancer cells" refers to any cells that exhibit uncontrolled growth in a tissue or organ of a multicellular organism. The term "breast cancer" is understood to mean any cancer or cancerous lesion associated with breast tissue or breast tissue cells and can include precursors to breast cancer, for example, atypical ductal hyperplasia or non-atypical hyperplasia. Cancer can be understood as a disease in which a primary tumor or multiple individual primary tumors exist, e.g. in the breast or breasts in case of breast cancer. The term "tumor" refers to an abnormal benign or malignant mass of tissue that is not inflammatory and possesses no physiological function.

As used herein, the term "subject" or "patient" refers to any animal (e.g., a mammal), including, but not limited to, humans, non-human primates, rodents, and the like. Typically, the terms "subject" and "patient" are used interchangeably herein in reference to a human subject. As used herein, the term "subject suspected of having cancer" refers to a subject that presents one or more symptoms indicative of a cancer (e.g., a noticeable lump or mass). A subject suspected of having cancer may also have one or more risk factors. A subject suspected of having cancer has generally not been tested for cancer. However, a "subject suspected of having cancer" encompasses an individual who has received an initial diagnosis (e.g., a CT scan showing a mass) but for whom the sub-type or stage of cancer is not known. The term further includes people who once had cancer (e.g., an individual in remission), and people who have cancer and are suspected to have a metastatic spread of the primary tumor.

In a preferred embodiment of the herein described invention, said subject is a mammal, preferably a human, more preferably a human patient diagnosed with cancer, or a human patient diagnosed with metastatic breast cancer. The invention further allows diagnosing not only breast cancer, but also metastatic breast cancer end even circulating tumor cell (CTC) positive breast cancer. Thus, the patient/subject of the invention may vary depending on which of the above stages of the disease is diagnosed, prognosed, stratified or tested. The person of skill is aware that, e.g., a patient already diagnosed with, e.g., metastatic breast cancer would not be tested for the general presence of, e.g., breast cancer as such. However, of course this shall not be interpreted as restriction of the scope of the invention as also already established diagnoses may be verified with any one of the herein described *in-vitro* methods of the invention.

In a particularly preferred embodiment, said subject is a mammal, such as a mouse, a rat, a guinea pig, a rabbit, a cat, a dog, a monkey, or a human, preferably a human, such as a human patient, more preferably a human patient suffering from a disease, condition or disorder and in need of a treatment.

The term "biological sample" as used herein refers to a sample that was obtained and may be assayed for any one of the biomarkers as disclosed with the present invention, or their gene expression. The biological sample can include a biological fluid (e.g., blood, cerebrospinal fluid, urine, plasma, serum), tissue biopsy, and the like. In some embodiments, the sample is a tissue sample, for example, tumor tissue, and may be fresh, frozen, or archival paraffin embedded tissue. Preferred samples for the purposes of the present invention are bodily fluids, in particular plasma samples.

In a preferred embodiment, the biological sample is a sample of a subject comprising a tissue sample or a body liquid sample, such as a sample of a group of cells from a tumor, a tumor tissue, an RNA sample, a DNA sample, a blood sample, a serum sample, a plasma sample, a urine sample, a lymph fluid sample, a pleural fluid sample, or a brain liquor sample, preferably a sample of a group of cells from a tumor or a tumor tissue.

The term "determining the level of" at least one first biomarker and at least one second biomarker in a sample, control or reference, as described herein, shall refer to the quantification of the presence of said at least one first biomarker and said at least one second biomarker in the tested sample. For example, the concentration of the first and second biomarker in said samples may be directly quantified via measuring the amount of protein/polypeptide/polysaccharide as present in the tested sample. Moreover, it is also possible to quantify the amount of the first and second biomarker directly via assessing the modified RNA nucleotide, for example by mass spectrometry. However, also possible is to quantify the amount of the first and second biomarker indirectly via assessing the gene expression of the encoding gene of the first and second biomarker, for example by quantification of the expressed mRNA encoding for the respective biomarker. How to determine the level of a particular biomarker is well known to the skilled artisan. The present invention shall not be restricted to any particular method for determining the level of a given biomarker, but shall encompass all means that allow for a quantification, or estimation, of the level of said first and second biomarker, either directly or indirectly. "Level" in the context of the present invention is therefore a parameter describing the absolute amount of a biomarker in a given sample, for example as absolute weight, volume, or molar amounts; or alternatively "level" pertains to the relative amounts, and preferably to the concentration of said biomarker in the tested sample, for example in mol/l, g/l, g/mol etc. In preferred embodiments the "level" refers to the concentration of the tested biomarkers in g/l.

The term "determining the mutation status" of at least one first and at least one second biomarker in a sample, control or reference, as described herein shall refer to analyzing the presence or absence of a mutation, such as a germline mutation and/or somatic mutation, for example in a gene of the pathway regulating N6-methyladenosine (m6A) mRNA modification, or in a gene of the pathway regulating 5-methoxycarbonylmethyl-2-thiouridine (mcm5s2U)-tRNA modification. How to determine the mutation status of a particular biomarker is well known to the skilled artisan. The present invention shall not be restricted to any particular method for determining a mutation status of a given biomarker, but shall encompass all means that allow for determination of the mutation status of said biomarker, either directly or indirectly. The presence or absence of a mutation may, for example, be determined by isolating nucleic acids obtained from cells, amplifying the nucleic acids and determining the presence or absence of the mutation in the amplified nucleic acids. In some of the embodiments, the presence or absence of the mutation is determined by allele-specific polymerase chain reaction (AS-PCR). As a standard value a corresponding property, in particular the base sequence, of a corresponding section of genomic DNA is suitable, which does not have the mutation, hereinafter also referred to as wild type, wild-type state or reference DNA. For example, genomic DNA from normal tissue, in particular healthy tissue, of the same individual is suitable as wild-type. This can be, for example, the preferred wild type with respect to the determination of a somatic mutation. As a wild-type, a reference genome can also be used. A suitable reference genome includes the human genome version of the Genome Reference Consortium Genome Reference Consortium Human Build 38 patch release 2 (GRCh38.p2) as of July 16, 2015. For example, a reference genome may be the preferred wild type for determining a germline mutation and/or somatic mutation.

A method of "determining the mutation status" of at least one first and at least one second biomarker of this invention may comprise the steps of amplifying a DNA sample obtained from, e.g. a patient or reverse-transcripting an RNA sample obtained from the patient into a DNA, amplifying the DNA, and analyzing the amplified DNA to determine at least one sequence abnormality with respect to a gene involved in the pathway regulating N6-methyladenosine (m6A) mRNA modification, and/or involved in the pathway regulating 5-methoxycarbonylmethyl-2-thiouridine (mem5s2U)-tRNA modification. Determination of a mutation status can, for example, be done by Sanger Sequencing. The methods of this invention may involve determining a single nucleotide polymorphism (SNP) and/or analysis of a single nucleotide mutation (i.e. a nucleotide substitution, insertion, deletion).

The term "determining the activity" of at least one biomarker in a sample, control or reference, as described herein shall refer to, for example, analyzing the enzymatic activity of at least one of the biomarkers of this invention, e.g. by performing an enzymatic assay of an enzyme or protein involved in the pathway regulating a m6A-mRNA modification, and/or involved in the pathway regulating a mcm5s2U-tRNA modification. According to this invention, the term "determining the activity" of at least one biomarker shall further include analyzing the phosphorylation state of a of an enzyme or protein involved in the pathway regulating a m6A-mRNA modification, and/or involved in the pathway regulating a mcm5s2U-tRNA modification, wherein the activity of the enzyme or protein is dependent upon at least one of the phosphorylation states of an amino acid of the particular enzyme or protein. How to determine the activity of a particular biomarker and/or the phosphorylation of an enzyme and/or protein is well known to the skilled artisan, and may, in one particular embodiment, involve western blotting or ELISA. The present invention shall, however, not be restricted to any particular method for determining the activity of a given biomarker, but shall encompass all means that allow for determination of the activity of said biomarker, either directly or indirectly.

The term "at least one" according to this invention shall include 1, 2, 3, 4, 5, 6, 7, 8, 9, 10, 11, 12, 13, 14, 15, 16, 17, 18, 19, 20, or any other number. For example, the term determining the level, mutation status, and/or activity of "at least one" first biomarker according to this invention shall include determining 1, 2, 3, 4, 5, 6, 7, 8, 9, 10, 11, 12, 13, 14, 15, 16, 17, 18, 19, 20, or any other number of possible first biomarkers, i.e. biomarkers indicative and/or selective for the status and/or activity of the pathway regulating N6-methyladenosine (m6A) mRNA modification in a cell. Further, the term determining the level, mutation status, and/or activity of "at least one" second biomarker according to this invention shall include determining 1, 2, 3, 4, 5, 6, 7, 8, 9, 10, 11, 12, 13, 14, 15, 16, 17, 18, 19, 20, or any other number of possible second biomarkers, i.e. biomarkers indicative and/or selective for the status and/or activity of the pathway regulating 5-methoxycarbonylmethyl-2-thiouridine (mcm5s2U)-tRNA modification in a cell. Also, the term determining "at least one" of the phosphorylation states of an amino acid of a particular enzyme or protein according to this invention shall include determining 1, 2, 3, 4, 5, 6, 7, 8, 9, 10, 11, 12, 13, 14, 15, 16, 17, 18, 19, 20, or any other number of possibly phosphorylated amino acids of the particular enzyme or protein.

A "biomarker" or "marker" in the context of the present invention refers to an organic biomolecule, particularly a polypeptide or polysaccharide, which is, for example, differentially present in a sample taken from patients having a certain condition as compared to a comparable sample taken from subjects who do not have said condition (e.g., negative diagnosis, normal or healthy subject). For example, a marker can be a polypeptide or polysaccharide (having a particular apparent molecular weight) which is present at an elevated or decreased level in samples of metastatic breast cancer patients compared to samples of patients with a negative diagnosis, or non-metastatic breast cancer patients. "Increase" of the level of a biomarker in a sample compared to a control shall, in preferred embodiments, refer to a statistically significant increase in preferred aspects of the invention. In alternative embodiments of the invention, certain biomarkers as disclosed herein may also be significantly decreased in the event of a particular disease, condition or disorder in a subject.

It is preferred that the analysis of a marker in step **(b)** and/or of a marker in step **(c)** of the *in-vitro* prognostic and/or *in-vitro* diagnostic method of the invention is characterized in that at least one of the tested markers has an apparent area under the curve (AUC) at 95% confidence interval (CI) of at least 60%, preferably at least 65% or more preferably at least 70%. How to determine the AUC is known to the skilled artisan. Alternatively or additionally, the biomarkers of the invention may be characterized by a sensitivity of at least 75%, preferably at least 80%, and a specificity of at least 40%, preferably at least 50%, more preferably at least 60%.

This invention is based on the discovery that a mcm5s2U modified tRNA enhances the decoding of m6A-modified mRNA at the wobble position, thereby regulating gene expression and cell differentiation. The inventors thus discovered a mechanism by which m6A connects nuclear mRNA processing to tRNA decoding, which represents a novel approach for prognosis, diagnosis, and/or monitoring of a disease, condition or disorder in a subject, and for therapeutic intervention in diseases, where gene expression is dysregulated by means of the m6A/mcm5s2U axis. The inventors found that decoding of m6A-modified mRNA can be facilitated by the tRNA anticodon modification mcm5s2U, thereby revealing an unanticipated link between the mRNA and tRNA epitranscriptomes. This system controls mRNA half-life in a translation-dependent manner and thus connects the exon-intron architecture of mRNAs to their turnover.

The inventors further discovered that cells balance the expression of m6A and mcm5s2U biogenesis machineries and failure to do so is associated with diseases, such as cancer, suggesting that the m6A/mcm5s2U axis has evolved as an epitranscriptomic mechanism to regulate gene expression and control cell differentiation. In healthy human tissues, the expression of biogenesis factors for m6A and mcm5s2U is highly correlated. Most importantly, CRISPR gene-dependency data shows that cancer cells that depend on m6A biogenesis factors also require mcm5s2U biogenesis factors and vice-versa. These results suggest that the m6A- and mcm5s2U-epitranscriptomes are intricately balanced, thus, modification of the tRNA anticodon with mcm5s2U is counteracting an imbalance of m6A, and vice versa.

To determine a gene, mRNA or protein involved in the biogenesis factors for m6A, a gene, mRNA or protein of the m6A methyltransferase complex (m6A-MTC), an m6A reader protein, and/or an m6A eraser protein is preferably analyzed. The m6A methyltransferase complex (m6A-MTC) comprises multiple protein components, such as METTL3, METTL14, WTAP, VIRMA, and RBM15 ZC3H13. Further, the m6A reader proteins YTHDF1 (YTH1), YTHDF2 (YTH2) and YTHDF3 (YTH3) can be analyzed to determine the level, mutation status, and/or activity of at least one first biomarker indicative and/or selective for the status and/or activity of the pathway regulating N6-methyladenosine (m6A) mRNA modification in a cell. In addition, the m6A demethylases FTO and ALKBH5 can be analyzed to determine the level, mutation status, and/or activity of at least one first biomarker indicative and/or selective for the status and/or activity of the pathway regulating N6-methyladenosine (m6A) mRNA modification in a cell.

Preferably, any of the proteins of the m6A-MTC, the m6A reader proteins, and the m6A demethylases can be analyzed to determine the level, mutation status, and/or activity of at least one first biomarker indicative and/or selective for the status and/or activity of the pathway regulating N6-methyladenosine (m6A) mRNA modification in a cell. In a further preferred embodiment, METTL3, METTL14, WTAP, VIRMA, RBM15, ZC3H13, YTHDF1, YTHDF2, YTHDF3, FTO, KIAA1429, CBLL1, RBM15, and/or ALKBH5 is analyzed to determine the level, mutation status, and/or activity of at least one first biomarker indicative and/or selective for the status and/or activity of the pathway regulating N6-methyladenosine (m6A) mRNA modification in a cell.

mcm5s2U biogenesis factors are required for pluripotency gene expression and stem cell maintenance [3,4], and defects in this pathway are linked to neurodegeneration, viral infection and cancer. Biogenesis of mcm5s2U is a multi-step process that involves the Elongator complex (consisting of ELP1/IKBKAP, ELP2, ELP3, ELP4, ELP5, and ELP6), the methyltransferase ALKBH8, MOCS3, URM1, and the CTU1-CTU2 thiolase complex. Therefore, proteins of the Elongator complex and the methyltransferase ALKBH8 can be analyzed to determine a gene, mRNA or protein involved in the biogenesis of factors for mcm5\s2U-tRNA. Further, the CTU1-CTU2 thiolase complex (i.e., CTU1, and/or CTU2) can be analyzed to determine the level, mutation status, and/or activity of at least one first biomarker indicative and/or selective for the status and/or activity of the pathway regulating N6-methyladenosine (m6A) mRNA modification in a cell. Further, MOCS3, and URM1, which are upstream of CTU1 and 2 in the mem5sU biogenesis pathway, can also be analyzed to determine the level, mutation status, and/or activity of at least one first biomarker indicative and/or selective for the status and/or activity of the pathway regulating N6-methyladenosine (m6A) mRNA modification in a cell.

To determine a gene, mRNA or protein involved in the biogenesis factors for 5-methoxycarbonylmethyl-2-thiouridine (mcm5s2U)-tRNA, a gene, mRNA or protein of the pathway regulating mcm5s2U-tRNA modification in a cell, preferably a gene, mRNA or protein such as ELP1/IKBKAP, ELP2, ELP3, ELP4, ELP5, ELP6, CTU1, CTU2, MOCS3, URM1, and/or ALKBH8 is analyzed.

In a particularly preferred embodiment said at least one first biomarker indicative and/or selective for the status and/or activity of the pathway regulating N6-methyladenosine (m6A) mRNA modification in a cell is selected from a gene, mRNA or protein of the m6A methyltransferase complex (m6A-MTC), an m6A reader protein, and an m6A eraser protein, and preferably is METTL3, METTL14, WTAP, VIRMA, RBM15, ZC3H13, YTHDF1, YTHDF2, YTHDF3, FTO, KIAA1429, CBLL1, RBM15, and/or ALKBH5.

Said at least one first biomarker indicative and/or selective for the status and/or activity of the pathway regulating N6-methyladenosine (m6A) mRNA modification in a cell can further be a signature panel comprising at least two, three, four, five, six, seven, eight, nine, ten, eleven, twelve, thirteen, or fourteen of said first biomarker selected from a gene, mRNA or protein of the m6A methyltransferase complex (m6A-MTC), an m6A reader protein, and an m6A eraser protein, such as selected from METTL3, METTL14, WTAP, VIRMA, RBM15, ZC3H13, YTHDF1, YTHDF2, YTHDF3, FTO, KIAA1429, CBLL1, RBM15, and ALKBH5.

In a further particularly preferred embodiment said at least one second biomarker indicative and/or selective for the status and/or activity of the pathway regulating 5-methoxycarbonylmethyl-2-thiouridine (mcm5s2U)-tRNA modification in a cell is selected from a gene, mRNA or protein of ELP1/IKBKAP, ELP2, ELP3, ELP4, ELP5, ELP6, CTU1, CTU2, MOCS3, URM1, and ALKBH8.

Said at least one second biomarker indicative and/or selective for the status and/or activity of the pathway regulating mcm5s2U-tRNA modification in a cell can further be a signature panel comprising at least two, three, four, five, six, seven, eight, nine, ten, or eleven of said second biomarker selected from a gene, mRNA or protein of the pathway regulating mcm5s2U-tRNA modification in a cell, such as selected from ELP1/IKBKAP, ELP2, ELP3, ELP4, ELP5, ELP6, CTU1, CTU2, MOCS3, URM1, and ALKBH8.

Yet another particularly preferred embodiment relates to said at least one second biomarker indicative and/or selective for the status and/or activity of the pathway regulating mcm5s2U-tRNA modification in a cell, wherein said second biomarker is selected from a gene, mRNA or protein of CTU1, CTU2, MOCS3, and URM1, or a combination thereof.

Said at least one second biomarker indicative and/or selective for the status and/or activity of the pathway regulating mcm5s2U-tRNA modification in a cell can further be a signature panel comprising at least two, or three, or all four of said second biomarkers selected from a gene, mRNA or protein of CTU1, CTU2, MOCS3, and URM1. Preferably, said panel comprising at least two, or three, or all four of said second biomarkers is selected from a combination of the biomarkers CTU1, and CTU2; CTU1, and MOCS3; CTU1, and URM1; CTU2, and MOCS3; CTU2, and URM1; MOCS3, and URM1; CTU1, CTU2, and MOCS3; CTU1, CTU2, and URM1; CTU1, MOCS3, and URM1; CTU2, MOCS3, and URM1; and CTU1, CTU2, MOCS3, and URM1. A particularly preferred embodiment relates to a panel comprising all four of the second biomarkes selected from a gene, mRNA or protein of CTU1, CTU2, MOCS3, and URM1. Thus, a preferred example consists of a panel of CTU1, CTU2, MOCS3, and URM1.

The skilled artisan will understand that numerous methods may be used to select a threshold or reference value for a particular marker or a plurality of markers. In diagnostic aspects, a threshold value may be obtained by performing the assay method on samples obtained from a population of patients having, for example, a certain type of cancer, and from a second population of subjects that do not have cancer. Alternatively, a threshold value may be obtained by performing the assay method on samples obtained from a population of patients having, for example, an aggressive type of cancer associated with a reduced time of progression free survival (PFS) and/or overall survival (OS), and from a second population of subjects that do have a rather mild form of cancer associated with an increased time of progression free survival (PFS) and/or overall survival (OS).

In the context of the above *in-vitro* method, an increase or a decrease of a differential level, mutation status, and/or activity of said first and/or second biomarker may be indicative for a reduced time of progression free survival (PFS) and/or overall survival (OS). If decreased levels of the biomarker are associated with poor prognosis, then also a decrease of said biomarker in said sample may be indicative for a reduced time of progression free survival (PFS) and/or overall survival (OS).

For prognostic or treatment monitoring applications, a population of patients, all of which have, for example, breast cancer, may be followed for the time period of interest (e.g., six months following diagnosis or treatment, respectively), and then dividing the population into multiple groups, for example two groups: a first group of subjects that progresses to an endpoint (e.g., recurrence of disease, and/or death); and a second group of subjects that did not progress to the end point (e.g., no recurrence of disease, and/or no death). These are used to establish "low risk" and "high risk" population values for the marker(s) measured, respectively. Other suitable endpoints include, but are not limited to, 5-year mortality rates or progression to metastatic disease.

Once these groups are established, one or more thresholds may be selected that provide an acceptable ability to predict prognostic risk, diagnosis, treatment success, etc. In practice, Receiver Operating Characteristic curves, or "ROC" curves, are typically calculated by plotting the value of a variable versus its relative frequency in two populations (called arbitrarily "disease" and "normal" or "low risk" and "high risk", for example). For any particular marker, a distribution of marker level for subjects with and without a disease may overlap. Under such conditions, a test does not absolutely distinguish "disease" and "normal" with 100% accuracy, and the area of overlap indicates where the test cannot distinguish "disease" and "normal." A threshold is selected, above which (or below which, depending on how a marker changes with the disease) the test is considered to be "positive" and below which the test is considered to be "negative." The area under the ROC curve (AUC) is a measure of the probability that the perceived measurement may allow correct identification of a condition.

Additionally, thresholds may be established by obtaining an earlier marker result from the same patient, to which later results may be compared. In some aspects, the individuals act as their own "control group." In markers that increase with disease severity or prognostic risk, an increase over time in the same patient can indicate a worsening of disease or a failure of a treatment regimen, while a decrease over time can indicate remission of disease or success of a treatment regimen.

In some embodiments, multiple thresholds or reference values may be determined. This can be the case in so-called "tertile," "quartile," or "quintile" analyses. In these methods, the "disease" and "normal" groups (or "low risk" and "high risk") groups can be considered together as a single population, and are divided into 3, 4, or 5 (or more) "bins" having equal numbers of individuals. The boundary between two of these "bins" may be considered "thresholds." A risk (of a particular prognosis or diagnosis for example) can be assigned based on which "bin" a test subject falls into.

All numeric values are herein assumed to be modified by the term "about," whether or not explicitly indicated. The term "about" generally refers to a range of numbers that one of skill in the art would consider equivalent to the recited value (i.e., having the same function or result). In many instances, the terms "about" may include numbers that are rounded to the nearest significant figure. In particularly preferred embodiments of the invention the term "about" may refer to a deviation of the respective numeric value of a maximum of 20% of the numerical value, however more preferred is 15%, 10%, 5%, even more preferred is 4%, 3%, 2%, and most preferred is 1%.

In a particularly preferred embodiment said disease, condition or disorder to be prognosed, diagnosed, and/or monitored is cancer, a viral disease, a cardiac disease, a neurodegenerative disease, a disease associated with oxidative stress, and/or other disease that shows a differential level, mutation status, and/or activity of the pathway regulating N6-methyladenosine (m6A) mRNA modification in a cell, and/or that show a differential level, mutation status, and/or activity of the pathway regulating 5-methoxycarbonylmethyl-2-thiouridine (mcm5s2U)-tRNA modification in a cell.

Further preferred is that said cancer is selected from breast cancer, melanoma, glioblastoma, endometrial cancer, Acute Myeloid Leukemia (AML), lung cancer, bladder cancer, hepatocellular cancer, medulloblastoma, kidney cancer, liver cancer, head and neck cancer, prostate cancer, pancreatic cancer, stomach cancer, colon cancer, thyroid cancer, esophageal cancer, brain cancer, colorectal cancer, gastric cancer, cervical cancer, ovarian cancer, cancer of the urinary tract, renal cancer, carcinoma, and a tumor that shows a differential level, mutation status, and/or activity of the pathway regulating m6A-mRNA modification in a cell, and/or regulating mcm5s2U-tRNA modification in a cell.

As used herein, the term "colorectal cancer" includes the well-accepted medical definition that defines colorectal cancer as a medical condition characterized by cancer of cells of the intestinal tract below the small intestine (i.e., the large intestine (colon), including the cecum, ascending colon, transverse colon, descending colon, sigmoid colon, and rectum). Additionally, as used herein, the term "colorectal cancer" also further includes medical conditions, which are characterized by cancer of cells of the duodenum and small intestine (jejunum and ileum).

As used herein, the terms "gastric cancer" or "stomach cancer" refer to cancers of the stomach. The most common types of gastric cancer are carcinomas, such as but not limited to, adenocarcinomas, affecting the epithelial cells of the stomach. Stomach cancers may additionally include, for example, sarcomas affecting the connective tissue of the stomach and blastomas affecting the blast tissue of the stomach.

The term "pancreatic cancer" according to this invention shall encompass benign or malignant forms of pancreatic cancer, as well as any particular type of cancer arising from cells of the pancreas (e.g., duct cell carcinoma, acinar cell carcinoma, papillary carcinoma, adenosquamous carcinoma, undifferentiated carcinoma, mucinous carcinoma, giant cell carcinoma, mixed type pancreatic cancer, small cell carcinoma, cystadenocarcinoma, unclassified pancreatic cancers, pancreatoblastoma, and papillary-cystic neoplasm, and the like.).

In a preferred embodiment of the invention, the cancer is preferably a cancer of epithelial origin. Epithelial cancers are, for example, epithelial cancer of the ovary, colon, lung, rectum, breast, prostate, pancreas, esophagus, bladder, liver, uterus, or brain.

In a further preferred embodiment of the invention, the determined level, mutation status, and/or activity of said first and said second biomarker in steps **(b)** and **(c)** are compared with a reference sample or a reference value in step **(d)** of the *in-vitro* method. Preferably, the reference sample or a reference value is a "cohort-intrinsic" reference value. In a particular embodiment, the signatures of tumor samples are binned into ntiles (e.g. into quartiles). Then, the survival of different ntiles (e.g. quartiles) is compared. A further embodiment of the invention relates to the *in-vitro* method of this invention, wherein a reference sample or a reference value in step **(d)** of the *in-vitro* method corresponds to a sample obtained from a healthy individual or obtained from a tumor-adjacent tissue from a subject suffering from a tumor disease.

A particularly preferred embodiment of the present invention pertains to an *in-vitro* method for the prognosis of a cancer patient, such as a breast cancer patient, comprising the steps of:
**(a)** Providing a biological sample of said subject,
**(b)** Determining the level, mutation status, and/or activity of at least one first biomarker indicative and/or selective for the status and/or activity of the pathway regulating N6-methyladenosine (m6A) mRNA modification in a cell in said biological sample,
**(c)** Determining the level, mutation status, and/or activity of at least one second biomarker indicative and/or selective for the status and/or activity of the pathway regulating 5-methoxycarbonylmethyl-2-thiouridine (mcm5s2U)-tRNA modification in a cell in said biological sample, and
**(d)** Comparing the determined level, mutation status, and/or activity of said first and said second biomarker in steps **(b)** and **(c)** with a reference sample or a reference value, wherein a differential level, mutation status, and/or activity of said first and/or second biomarker in the biological sample from said subject compared to said reference sample or reference value is indicative for a reduced time of progression free survival (PFS) and/or overall survival (OS) of a patient suffering from a disease, condition or disorder.

One preferred embodiment, which can be combined with any of the other preferred embodiments of the invention, pertains to any of the above described *in-vitro* methods, wherein the level, mutation status, and/or activity of said at least one first biomarker and said at least one second biomarker is determined by means of a detection method comprising mRNA analysis, DNA analysis, protein analysis, and/or a method for determining a single nucleotide polymorphism (SNP) in a target nucleic acid sequence.

In all aspects and embodiments of the present invention it may be preferred that the level of said at least one biomarker in said sample is determined by means of a nucleic acid detection method or an immunological detection method. However, nucleic acid detection methods are only applicable where an expressed protein is the biomarker. Generally, all means shall be comprised by the present invention which allow for a quantification of the expression, mutation status, and/or activity of such proteins. Therefore also promoter analysis, and procedures assessing the epigenetic status of a gene locus encoding a protein biomarker of the invention are comprised by the herein described invention. Alternatively, the presence or expression of enzymes regulating the metabolism of a protein may be assessed, which is indicative of the presence or level of said protein.

Detection methods that are preferred in context of the herein described invention for determining the level, mutation status, and/or activity of said at least one first biomarker and of said at least second first biomarker in said sample shall include means of a detection method selected from the group consisting of mass spectrometry, mass spectrometry immunoassay (MSIA), antibody-based protein chips, 2-dimensional gel electrophoresis, stable isotope standard capture with anti-peptide antibodies (SISCAPA), high-performance liquid chromatography (HPLC), western blot, cytometry bead array (CBA), protein immuno-precipitation, radio immunoassay, ligand binding assay, and enzyme-linked immunosorbent assay (ELISA), preferably wherein said protein detection method is ELISA. Suitable alternative detection methods for determination of a biomarker of the invention are known to the skilled artisan.

In a particularly preferred embodiment, the detection method used for determining the level, mutation status, and/or activity of said at least one first biomarker and of said at least second first biomarker in said sample is selected from a detection method comprising mRNA analysis, DNA analysis, protein analysis, and/or a method for determining a single nucleotide polymorphism (SNP) in a target nucleic acid sequence, such as a polymorphic array comprising a set of single nucleotide polymorphisms (SNPs), for example, an Affymetrix GeneChip, polymerase chain reaction (PCR), real-time quantitative PCR (qPCR), nanoString nCounter, enzyme-linked immunosorbent assay (ELISA), mass spectrometry, mass spectrometry immunoassay (MSIA), antibody-based protein chips, 2-dimensional gel electrophoresis, stable isotope standard capture with anti-peptide antibodies (SISCAPA), high-performance liquid chromatography (HPLC), genetic testing, western blot, cytometry bead array (CBA), radio immunoassay, and immunohistochemical staining, preferably wherein said detection method is a polymorphic array for determining a single nucleotide polymorphism (SNP) in a target nucleic acid sequence, and/or real-time quantitative PCR (qPCR).

Yet another preferred embodiment of this invention relates to an *in-vitro* method, wherein a differential level, mutation status, and/or activity of said at least one first biomarker and/or said at least one second biomarker in the biological sample from said subject compared to said reference sample or reference value is indicative for a reduced time of progression free survival (PFS) and/or overall survival (OS) of the patient suffering from the disease, condition or disorder, and wherein said reference sample or reference value is a cohort-intrinsic reference sample or reference value, wherein the level, mutation status, and/or activity of said at least one first biomarker and/or said at least one second biomarker in the reference sample or reference value compared to the biological sample from said subject is indicative for an increased time of progression free survival (PFS) and/or overall survival (OS).

A further preferred embodiment of this invention relates an *in-vitro* method, wherein a differential level, mutation status, and/or activity of said at least one first biomarker and/or said at least one second biomarker in the biological sample from said subject compared to said reference sample or reference value is indicative for the presence of cancer in said subject, preferably wherein said cancer is melanoma, glioblastoma, breast cancer, and/or endometrial cancer.

An additionally preferred embodiment of this invention relates to an *in-vitro* method, wherein a differential ratio of (i) a level of at least one gene, mRNA or protein regulating an m6A-mRNA modification compared to (ii) a gene, mRNA or protein regulating a mcm5s2U-tRNA modification is indicative for a disease, condition or disorder.

In a particularly preferred embodiment of the present invention, a relative level of the level, mutation status, and/or activity of at least one first biomarker indicative and/or selective for the status and/or activity of the pathway regulating m6A mRNA modification in a cell, to the level, mutation status, and/or activity of at least one first biomarker indicative and/or selective for the status and/or activity of the pathway regulating mcm5s2U-tRNA modification in a cell is determined by a method comprising the steps of:
**(a)** measuring i) at least one first biomarker selected from a gene, mRNA or protein of the m6A methyltransferase complex (m6A-MTC), an m6A reader protein, and an m6A eraser protein, and preferably is METTL3, METTL14, WTAP, VIRMA, RBM15, ZC3H13, YTHDF1, YTHDF2, YTHDF3, FTO, KIAA1429, CBLL1, RBM15, and/or ALKBH5s; and ii) at least one second biomarker is selected from a gene, mRNA or protein of ELP1/IKBKAP, ELP2, ELP3, ELP4, ELP5, ELP6, CTU1, CTU2, MOCS3, URM1, and/or ALKBH8, and
**(b)** determining said relative level.

In a particularly preferred embodiment, said at least one second biomarker (ii) as measured in (a) comprises a panel of CTU1, CTU2, MOCS3, and URM1, and/or said at least one second biomarker (ii) consists of a panel of CTU1, CTU2, MOCS3, and URM1.

A further preferred embodiment of the present invention relates to the *in-vitro* method of this invention, wherein the differential level, mutation status, and/or activity of said first and/or second biomarker in the biological sample from said subject compared to said reference sample or reference value is indicative for any one of the following:
**(a)** a disease, condition or disorder associated with a higher level of m6A-modified mRNA and a lower level of mcm5s2U-modified tRNA compared to a healthy control,
**(b)** a disease, condition or disorder associated with a higher level of m6A-modified mRNA and a unchanged/normal level of mcm5s2U-modified tRNA compared to a healthy control,
**(c)** a disease, condition or disorder associated with a higher level of m6A-modified mRNA and a higher level of mcm5s2U-modified tRNA compared to a healthy control,
**(d)** a disease, condition or disorder associated with a lower level of m6A-modified mRNA and a higher level of mcm5s2U-modified tRNA compared to a healthy control,
**(e)** a disease, condition or disorder associated with a lower level of m6A-modified mRNA and a unchanged/normal level of mcm5s2U-modified tRNA compared to a healthy control,
**(f)** a disease, condition or disorder associated with a lower level of m6A-modified mRNA and a lower level of mcm5s2U-modified tRNA compared to a healthy control,
**(g)** a disease, condition or disorder associated with a unchanged/normal level of m6A-modified mRNA and a lower level of mcm5s2U-modified tRNA compared to a healthy control, or
**(h)** a disease, condition or disorder associated with a unchanged/normal level of m6A-modified mRNA and a higher level of mcm5s2U-modified tRNA compared to a healthy control.

In an additionally preferred embodiment of this invention, the healthy control is a comparable sample of a healthy subject, or is a reference value derived therefrom, wherein the healthy subject is known not to suffer from a disease, condition or disorder associated with a differential level of m6A-modified mRNA and/or mcm5s2U-modified tRNA.

In a further preferred embodiment of the invention, the healthy control is derived from a "cohort-intrinsic" reference value. In a particular embodiment, the signatures of tumor samples are binned into ntiles (e.g. into quartiles). Then, the different ntiles (e.g. quartiles) are compared. A further embodiment of the invention relates to any one of the methods of this invention, wherein a healthy control is defined as the ntile of the cohort showing the least symptoms of the disease and/or showing the best survival rate.

In an additionally preferred embodiment of this invention, if the determined differential level, mutation status, and/or activity of said first and/or second biomarker in the biological sample from said subject is (i) such that the result is indicative for a lower level of m6A-modified mRNA and a higher level of mem5s2U-modified tRNA, the time of progression free survival (PFS) and/or overall survival (OS) is reduced compared to a subject not showing such result, and/or (ii) such that the result is indicative for a higher level of m6A-modified mRNA and a lower level of mcm5s2U-modified tRNA, the time of progression free survival (PFS) and/or overall survival (OS) is prolonged compared to a subject not showing such result.

A further preferred embodiment of the present invention relates to the *in-vitro* method of this invention, wherein if the determined differential level, mutation status, and/or activity of said first and/or second biomarker in the biological sample from said subject is such that the result is indicative for a disease, condition or disorder associated with a lower level of m6A-modified mRNA and a higher level of mcm5s2U-modified tRNA (see **(d)** in the method above), for a disease, condition or disorder associated with a lower level of m6A-modified mRNA and an unchanged/normal level of mcm5s2U-modified tRNA (see **(e)** in the method above), or for a disease, condition or disorder associated with an unchanged/normal level of m6A-modified mRNA and a higher level of mcm5s2U-modified tRNA (see **(h)** in the method above), said disease condition or disorder is a cancer disease selected from bladder cancer, breast cancer, esophagus cancer, lung cancer, and thyroid cancer.

An additionally preferred embodiment of this invention is characterized in that if the determined differential level, mutation status, and/or activity of said first and/or second biomarker in the biological sample from said subject is such that the result is indicative for a disease, condition or disorder associated with a higher level of m6A-modified mRNA and a lower level of mcm5s2U-modified tRNA (see **(a)** in the method above), for a disease, condition or disorder associated with a higher level of m6A-modified mRNA and an unchanged/normal level of mcm5s2U-modified tRNA (see **(b)** in the method above), or for a disease, condition or disorder associated with a unchanged/normal level of m6A-modified mRNA and a lower level of mcm5s2U-modified tRNA (see **(g)** in the method above), said disease condition or disorder is a cancer disease selected from kidney cancer, liver cancer, and stomach cancer.

Yet another aspect of this invention relates to an *in-vitro* method of stratifying a patient for a disease, condition or disorder, said *in-vitro* method comprising, as a first step, identifying a differential level, mutation status, and/or activity of at least one first and/or at least one second biomarker in the biological sample from said patient compared to a reference sample or reference value, and, as a second step, deciding in favor of or against said treatment based on said differential level, mutation status, and/or activity, wherein said differential level, mutation status, and/or activity is identified by using a method comprising the steps of:
**(a)** Providing a biological sample of said patient,
**(b)** Determining the level, mutation status, and/or activity of at least one first biomarker indicative and selective for the status and/or activity of the pathway regulating N6-methyladenosine (m6A) mRNA modification in a cell in said biological sample,
**(c)** Determining the level, mutation status, and/or activity of at least one second biomarker indicative and selective for the status and/or activity of the pathway regulating 5-methoxycarbonylmethyl-2-thiouridine (mcm5s2U)-tRNA modification in a cell in said biological sample, and
**(d)** Comparing the determined level, mutation status, and/or activity of said first and second biomarker in steps **(b)** and **(c)** with a reference sample or a reference value.

The term "stratification" for the purposes of this invention shall refer to the advantage that the *in-vitro* method according to the invention renders decisions for the treatment and therapy of the patient possible, whether it is the hospitalization of the patient, the use, effect and/or dosage of one or more drugs, a therapeutic measure or the monitoring of a course of the disease and the course of therapy or etiology or classification of a disease, e.g., into a new or existing subtype or the differentiation of diseases and the patients thereof. Particularly with regard to breast cancer, "stratification" means in this context a classification of a breast cancer disease of an individual patient with regard of the metastatic status, or the presence or absence of circulating tumor cells. Also, the term "stratification" covers in particular the risk stratification with the prognosis of an outcome of a negative health event. In a particularly preferred embodiment, a condition, disorder, or disease, such as cancer, is stratified by the mcm5s2U pathway status to predict sensitivity to perturbations of m6A biogenesis by m6A targeting compounds. Additionally preferred is a *in-vitro* method, wherein a condition, disorder, or disease, such as cancer, is stratified by analyzing the expression and/or mutation status of the mcm5s2U pathway to predict sensitivity to perturbations of m6A biogenesis by m6A targeting compounds.

Yet another preferred embodiment relates to an *in-vitro* method of stratification of cancer patients into responders and non-responders to a therapy with m6A targeting compounds. Particularly preferred cancers to be stratified are AML and glioblastoma, since previous results show that AML and glioblastoma show variable responses to perturbation of the m6A pathway. Thus, the mcm5s2U pathway can be used to predict outcome of a therapy with m6A targeting compounds.

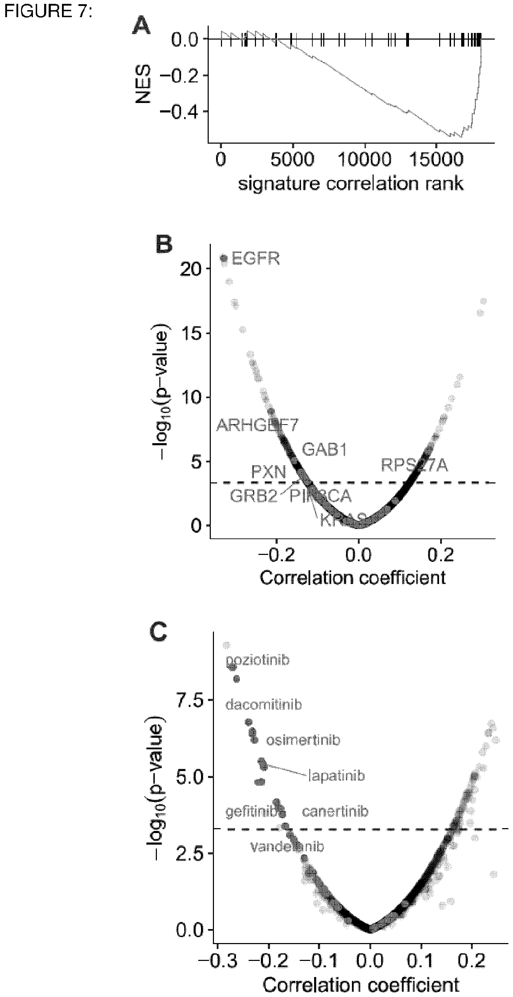

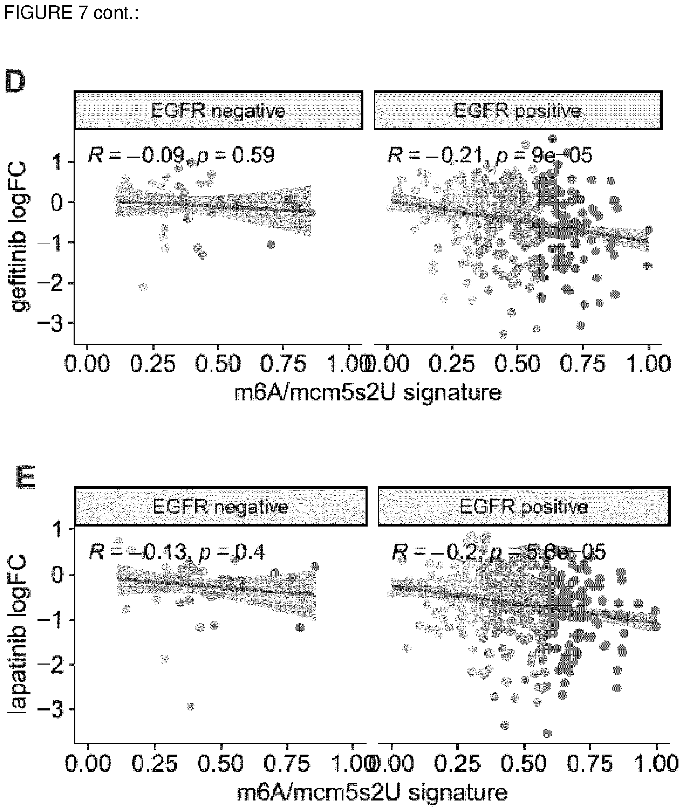

Importantly, the present invention can be used to predict the response of a patient to a treatment. The inventors observed that a high m⁶A/mcm⁵s²U signature correlates with increased sensitivity to EGFR-targeting tyrosine kinase inhibitors (TKIs) including lapatinib and gefitinib (Figure 7). As such, the combined m6A/mcm5s2U signature serves as a biomarker for the sensitivity to EGFR inhibition. Moreover, the combined m6A/mcm5s2U signature can be used as a biomarker to predict treatment success in a cancer patient. In one embodiment, the cancer patient is treated with EGFR-targeting tyrosine kinase inhibitors (TKIs). However, the present invention is not limited to predicting treatment success with EGFR-targeting TKIs. Instead, the combined m6A/mcm5s2U signature can be used as a biomarker to predict treatment success of a patient with any kind of treatment. In one embodiment, the treatment whose success shall be predicted is targeting the epitranscriptome.

An additionally preferred embodiment of this invention relates to the above-described *in-vitro* method of stratifying a patient for a treatment, wherein (i) said at least one first biomarker is selected from a gene, mRNA or protein of the m6A methyltransferase complex (m6A-MTC), an m6A reader protein, and an m6A eraser protein, and preferably is METTL3, METTL14, WTAP, VIRMA, RBM15, ZC3H13, YTHDF1, YTHDF2, YTHDF3, FTO, KIAA1429, CBLL1, RBM15, and/or ALKBH5s; and/or (ii) wherein said at least one second biomarker is selected from a gene, mRNA or protein of ELP1/IKBKAP, ELP2, ELP3, ELP4, ELP5, ELP6, CTU1, CTU2, MOCS3, URM1, and/or ALKBH8.

Particularly preferred is the above method, wherein said at least one second biomarker (ii) comprises a panel of CTU1, CTU2, MOCS3, and URM1, and/or wherein said at least one second biomarker (ii) consists of a panel of CTU1, CTU2, MOCS3, and URM1.

A further preferred embodiment of the present invention relates to the above *in-vitro* method of stratifying a patient for a treatment, wherein based on the differential level, mutation status, and/or activity of said first and/or second biomarker in the biological sample from said subject compared to said reference sample or value in **(d),** it is decided in favor of enhancing or inhibiting the level, mutation status, and/or activity of the pathway regulating N6-methyladenosine (m6A) mRNA modification, and/or enhancing or inhibiting the level, mutation status, and/or activity of the pathway regulating 5-methoxycarbonylmethyl-2-thiouridine (mem5s2U)-tRNA modification.

One preferred embodiment of the invention pertains to the above described *in-vitro* method of stratifying a patient for a treatment, wherein said treatment is a treatment targeting an m6A-mRNA modification in a cell and/or an mcm5s2U-tRNA modification in a cell, preferably wherein said treatment inhibits or activates the m6A methyltransferase complex (m6A-MTC), an m6A reader protein, an m6A eraser protein, and/or the elongator complex.

In an additionally preferred embodiment of this invention, if the differential level, mutation status, and/or activity of said first and/or second biomarker in the biological sample from said subject compared to said reference sample or value in **(d)** is at least 1%, preferably at least 5%, more preferably at least 10%, even more preferably at least 15%, and most preferably at least 20% higher or lower compared to said reference sample or reference value, it is decided in favor of said treatment.

However, the absolute concentration levels strongly depend on the sample handling and quantification method used. The above levels apply for a sample handling corresponding to the methods used in context of the examples. Therefore, the skilled in the art appreciates that without being confronted by undue experimentation, new reference values must be determined in accordance with a change of a sample treatment protocol.

The object of the present invention is furthermore solved by an *in-vitro* method for evaluating the treatment success of a patient who received a treatment, the *in-vitro* method comprising the steps of:
(a) Providing a biological sample of said patient who received a treatment,
(b) Determining the level, mutation status, and/or activity of at least one first biomarker indicative and selective for the status and/or activity of the pathway regulating N6-methyladenosine (m6A) mRNA modification in a cell in said biological sample,
**(c)** Determining the level, mutation status, and/or activity of at least one second biomarker indicative and selective for the status and/or activity of the pathway regulating 5-methoxycarbonylmethyl-2-thiouridine (mem5s2U)-tRNA modification in a cell in said biological sample, and
**(d)** Comparing the determined level, mutation status, and/or activity of said first and second biomarker in steps **(b)** and **(c)** with a reference sample or a reference value,
wherein a decrease or an increase of the differential level, mutation status, and/or activity of said first and/or second biomarker in the biological sample from said subject compared to said reference sample or reference value is an indication for the patient's response to said treatment.

In yet another preferred embodiment, an *in-vitro* method for evaluating the treatment success of a patient who received a treatment is preferred, wherein a decrease or an increase of at least 10%, preferably at least 20%, more preferably 25%, even more preferably 30%, and most preferably 35% to 45% of said level, mutation status, and/or activity of said first and/or second biomarker in the biological sample from said subject compared to said reference sample or reference value is an indication for the patient's response to said treatment.

A further preferred embodiment of the present invention relates to the *in-vitro* method of this invention, wherein (i) said at least one first biomarker is selected from a gene, mRNA or protein of the m6A methyltransferase complex (m6A-MTC), an m6A reader protein, and an m6A eraser protein, and preferably is METTL3, METTL14, WTAP, VIRMA, RBM15, ZC3H13, YTHDF1, YTHDF2, YTHDF3, FTO, KIAA1429, CBLL1, RBM15, and/or ALKBH5s; and/or (ii) wherein said at least one second biomarker is selected from a gene, mRNA or protein of ELP1/IKBKAP, ELP2, ELP3, ELP4, ELP5, ELP6, CTU1, CTU2, MOCS3, URM1, and/or ALKBH8.

The "reference" in the context of the here described aspect of the invention preferably corresponds to the level of said of at least one biomarker in a provided biological sample obtained from said patient before receiving said treatment. Thereby, the reference corresponds to an untreated control. In an additionally preferred embodiment, said reference corresponds to the ratio of the level, mutation status, and/or activity of said first biomarker to the level, mutation status, and/or activity of said second biomarker in a provided biological sample obtained from said patient before receiving said treatment.

A further preferred embodiment of the present invention relates to the *in-vitro* method of this invention, wherein said treatment is a treatment targeting an m6A-mRNA modification in a cell and/or an mcm5s2U-tRNA modification in a cell, preferably wherein said treatment inhibits or activates the m6A methyltransferase complex (m6A-MTC), an m6A reader protein, an m6A eraser protein, and/or the elongator complex.

In an additionally preferred embodiment of this invention, said disease, condition or disorder is preferably treated by adjusting the level of mcm5s2U to m6A, i.e. the mcm5s2U to m6A ratio, characterized in that a mcm5s2U-modified tRNA enhances the decoding of m6A-modified mRNA, thereby regulating gene expression and cell differentiation.

. All methods of the herein described invention are *in-vitro* methods, preferably in-*vitro* methods that do not comprise any method steps performed at the human or animal body.

A further preferred embodiment of the present invention relates to the *in-vitro* method of this invention, wherein said disease, condition or disorder is selected from cancer, a viral disease, a cardiac disease, a neurodegenerative disease, a disease associated with oxidative stress, and other diseases that show a differential level, mutation status, and/or activity of the pathway regulating N6-methyladenosine (m6A) mRNA modification in a cell, and/or that show a differential level, mutation status, and/or activity of the pathway regulating 5-methoxycarbonylmethyl-2-thiouridine (mem5s2U)-tRNA modification in a cell.

In an additionally preferred embodiment of this invention, said cancer is selected from melanoma, glioblastoma, breast cancer, endometrial cancer, Acute Myeloid Leukemia (AML), lung cancer, bladder cancer, hepatocellular cancer, medulloblastoma, kidney cancer, liver cancer, head and neck cancer, prostate cancer, pancreatic cancer, stomach cancer, colon cancer, thyroid cancer, esophageal cancer, brain cancer, colorectal cancer, gastric cancer, cervical cancer, ovarian cancer, cancer of the urinary tract, renal cancer, carcinoma, and a tumor that shows a differential level, mutation status, and/or activity of the pathway regulating m6A-mRNA modification in a cell, and/or regulating mcm5s2U-tRNA modification in a cell, preferably wherein said cancer is melanoma, glioblastoma, breast cancer, endometrial cancer, Acute Myeloid Leukemia (AML), lung cancer, bladder cancer, hepatocellular cancer, medulloblastoma, a tumor that shows a differential level, mutation status, and/or activity of the pathway regulating m6A-mRNA modification in a cell, and/or regulating mcm5s2U-tRNA modification in a cell.

In a **second aspect,** the invention pertains to the use of a kit in an *in-vitro* method for the prognosis, diagnosis, and/or monitoring of a disease, condition or disorder in a subject as defined the claims, the kit comprising means for quantifying the level, mutation status, and/or activity of the at least one m6A-mRNA modification biomarker indicative and/or selective for the status and/or activity of the pathway regulating m6A-mRNA modification in a cell, and/or for quantifying the level, mutation status, and/or activity of the at least one mcm5s2U-tRNA anticodon modification biomarker indicative and/or selective for the status and/or activity of the pathway regulating mcm5s2U-tRNA anticodon modification in a cell.

A further preferred embodiment of the present invention relates to the kit of this invention, wherein (i) said at least one first biomarker is selected from a gene, mRNA or protein of the m6A methyltransferase complex (m6A-MTC), an m6A reader protein, and an m6A eraser protein, and preferably is METTL3, METTL14, WTAP, VIRMA, RBM15, ZC3H13, YTHDF1, YTHDF2, YTHDF3, FTO, KIAA1429, CBLL1, RBM15, and/or ALKBH5s; and/or (ii) wherein said at least one second biomarker is selected from a gene, mRNA or protein of ELP1/IKBKAP, ELP2, ELP3, ELP4, ELP5, ELP6, CTU1, CTU2, MOCS3, URM1, and/or ALKBH8.

Particularly preferred is the above kit, wherein said at least one second biomarker (ii) comprises a panel of CTU1, CTU2, MOCS3, and URM1, and/or wherein said at least one second biomarker (ii) consists of a panel of CTU1, CTU2, MOCS3, and URM1.

One preferred embodiment of the invention pertains to the above described kit, comprising one or more antibodies, derivatives, or antigenic fragments thereof, for the detection of (i) at least one of a protein of the m6A methyltransferase complex (m6A-MTC), an m6A reader protein, and an m6A eraser protein, preferably of METTL3, METTL14, WTAP, VIRMA, RBM15, ZC3H13, YTHDF1, YTHDF2, YTHDF3, FTO, KIAA1429, CBLL1, RBM15, and/or ALKBH5s; and/or comprising one or more antibodies, derivatives, or antigenic fragments thereof, for the detection of (ii) at least one of ELP1/IKBKAP, ELP2, ELP3, ELP4, ELP5, ELP6, CTU1, CTU2, MOCS3, URM1, and/or ALKBH8.

The kits of the invention may have many applications. For example, the kits can be used to differentiate if a subject has a certain disease, condition or disorder, such as cancer, in particular breast cancer, such as metastatic breast cancer, or CTC positive breast cancer, or has a negative diagnosis, thus aiding a certain diagnosis.

Optionally, the kit can further comprise instructions for suitable operational parameters in the form of a label or a separate insert. For example, the kit may have standard instructions informing a consumer how to wash the probe after a sample of plasma is contacted on the probe.

In another embodiment, a kit comprises (a) an antibody that specifically binds to a marker; and (b) a detection reagent. Such kits can be prepared from the materials described above, and the previous discussion regarding the materials (e.g., antibodies, detection reagents, immobilized supports, etc.) is fully applicable to this section and need not be repeated.

In either embodiment, the kit may optionally further comprise a standard or control information so that the test sample can be compared with the control information standard to determine if the test amount of a marker detected in a sample is a diagnostic amount consistent with a diagnosis of a disease, condition or disorder, such as cancer, preferably breast cancer. Such antibodies are known in the art and are commercially available.

The terms "of the [present] invention", "in accordance with the invention", "according to the invention" and the like, as used herein are intended to refer to all aspects and embodiments of the invention described and/or claimed herein.

As used herein, the term "comprising" is to be construed as encompassing both "including" and "consisting of", both meanings being specifically intended, and hence individually disclosed embodiments in accordance with the present invention. Where used herein, "and/or" is to be taken as specific disclosure of each of the two specified features or components with or without the other. For example, "A and/or B" is to be taken as specific disclosure of each of (i) A, (ii) B and (iii) A and B, just as if each is set out individually herein. In the context of the present invention, the terms "about" and "approximately" denote an interval of accuracy that the person skilled in the art will understand to still ensure the technical effect of the feature in question. The term typically indicates deviation from the indicated numerical value by ±20%, ±15%, ±10%, and for example ±5%. As will be appreciated by the person of ordinary skill, the specific such deviation for a numerical value for a given technical effect will depend on the nature of the technical effect. For example, a natural or biological technical effect may generally have a larger such deviation than one for a man-made or engineering technical effect. As will be appreciated by the person of ordinary skill, the specific such deviation for a numerical value for a given technical effect will depend on the nature of the technical effect. For example, a natural or biological technical effect may generally have a larger such deviation than one for a man-made or engineering technical effect. Where an indefinite or definite article is used when referring to a singular noun, e.g. "a", "an" or "the", this includes a plural of that noun unless something else is specifically stated.

It is to be understood that application of the teachings of the present invention to a specific problem or environment, and the inclusion of variations of the present invention or additional features thereto (such as further aspects and embodiments), will be within the capabilities of one having ordinary skill in the art in light of the teachings contained herein.

Unless context dictates otherwise, the descriptions and definitions of the features set out above are not limited to any particular aspect or embodiment of the invention and apply equally to all aspects and embodiments which are described.

### BRIEF DESCRIPTION OF THE FIGURES AND SEQUENCES

The figures show:

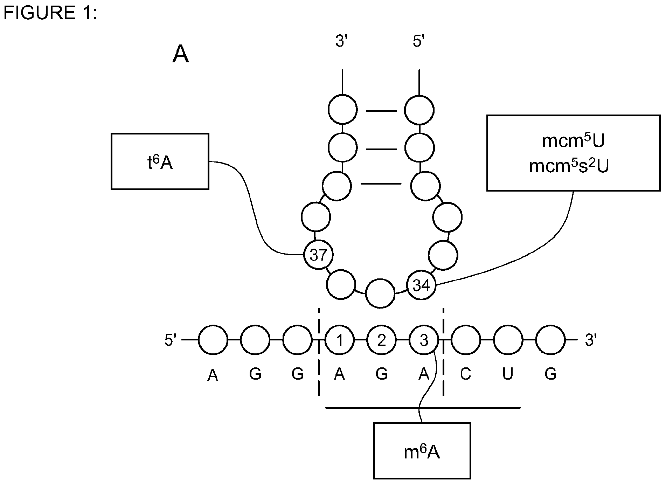

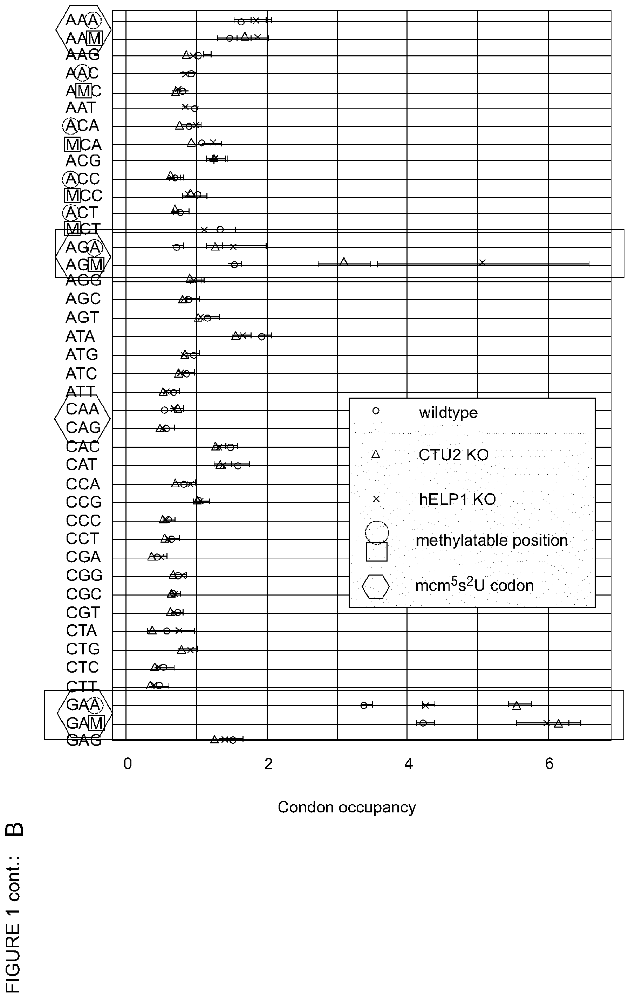

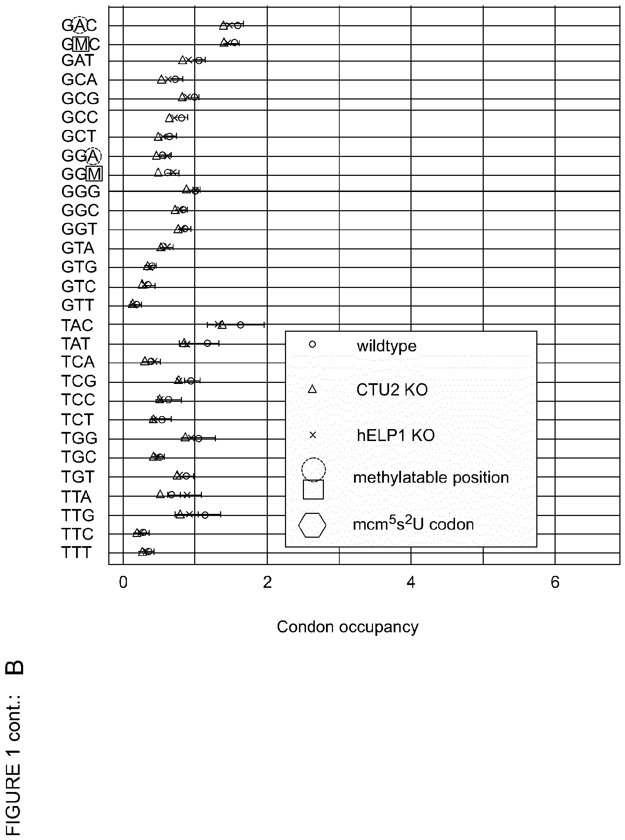

Figure 1 shows mcm5s2U modulates decoding of m6A-modified codons. A. Schematic of modified nucleotides in the codon and the tRNA anticodon loop. Spheres are nucleotides of mRNA (bottom) and tRNA (top). Modifications at tRNA positions 34 and 37 are indicated. m6A at codon position 3 is indicated, and the DRACH motif (AGACU) is highlighted by a line. B. Codon A-site occupancies as determined by ribosome profiling in wildtype HEK293 cells (circle) or cells in which CTU2 (triangle) or ELP1/IKBKAP (cross) was knocked out. Codons decoded by a mcm5s2U-modified tRNA are indicated by boxes. Methylatable adenosines are indicated by spheres.

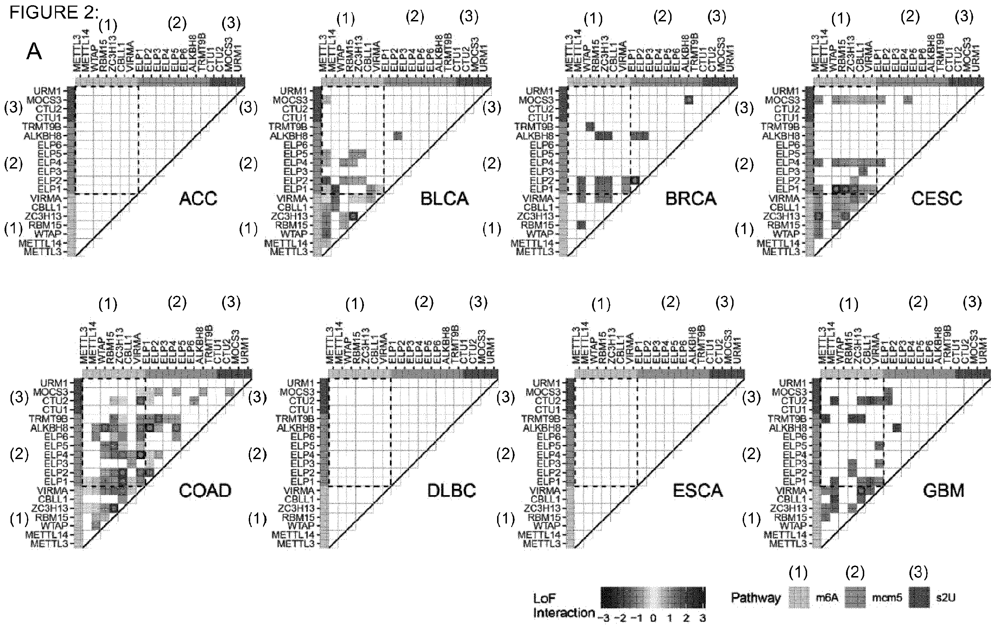

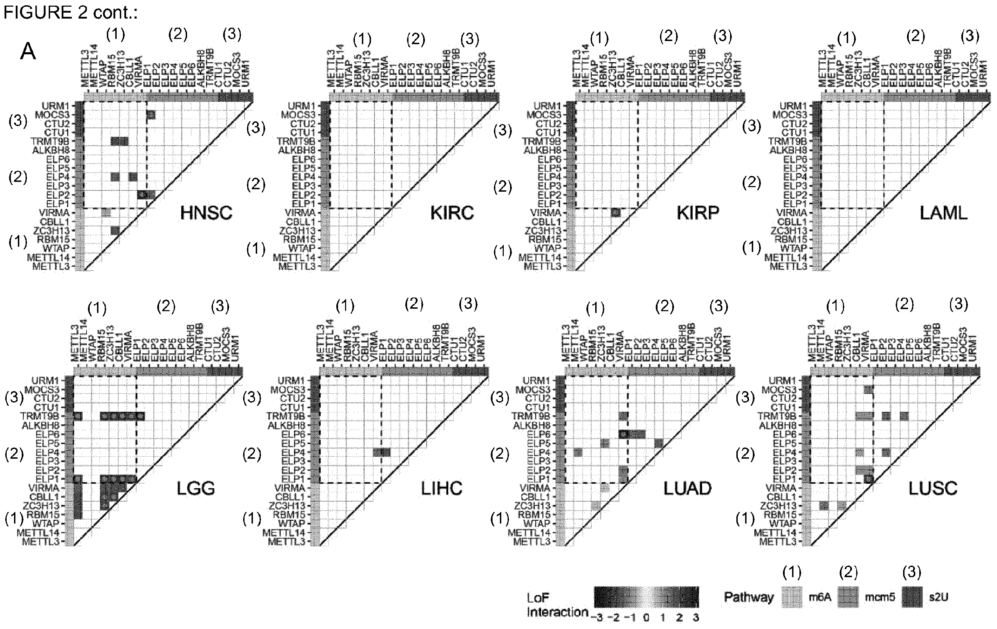

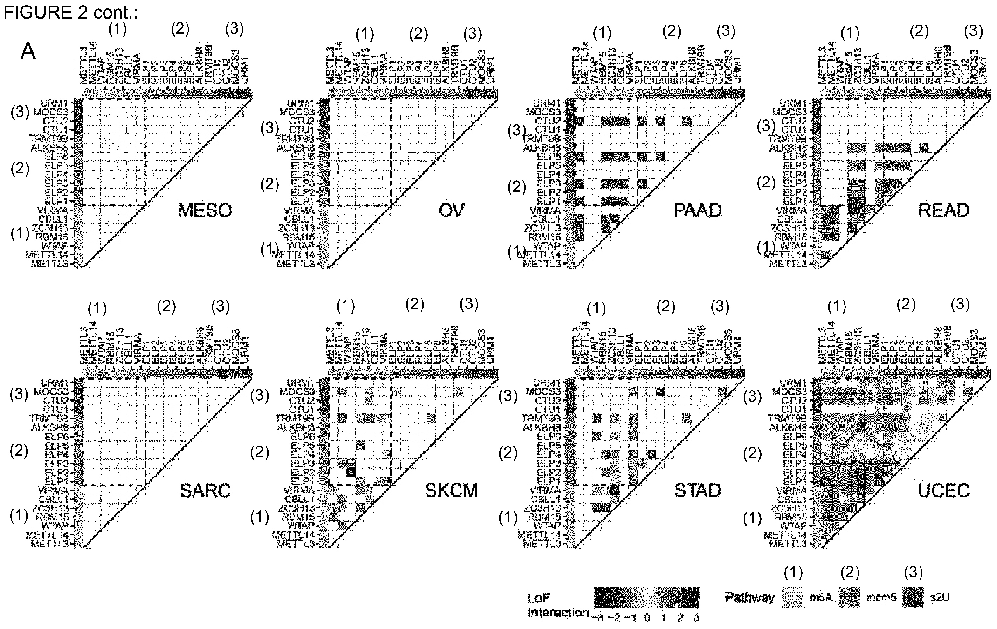

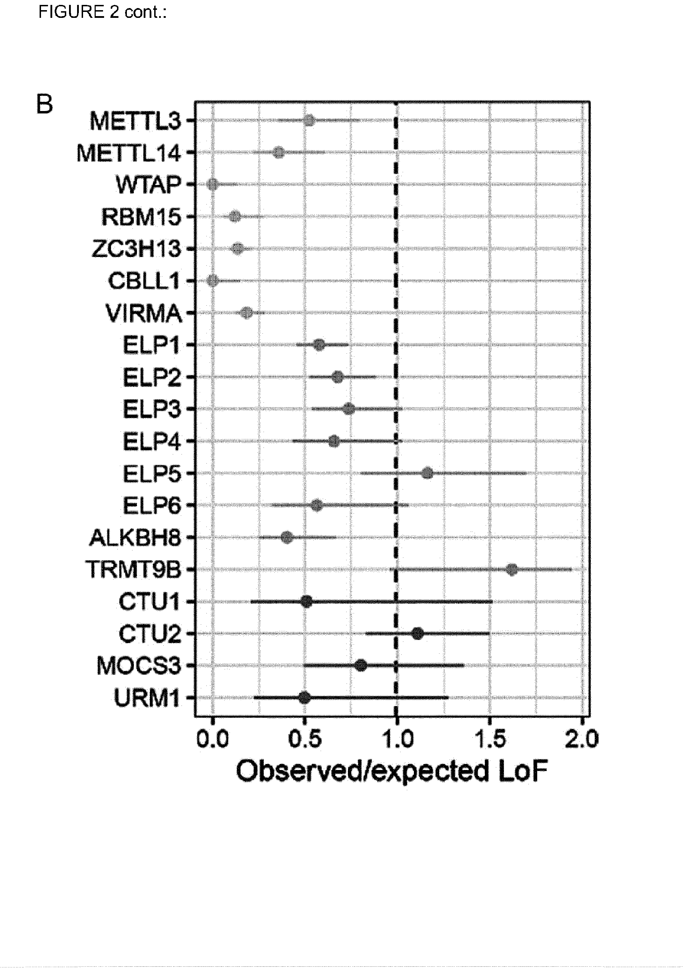

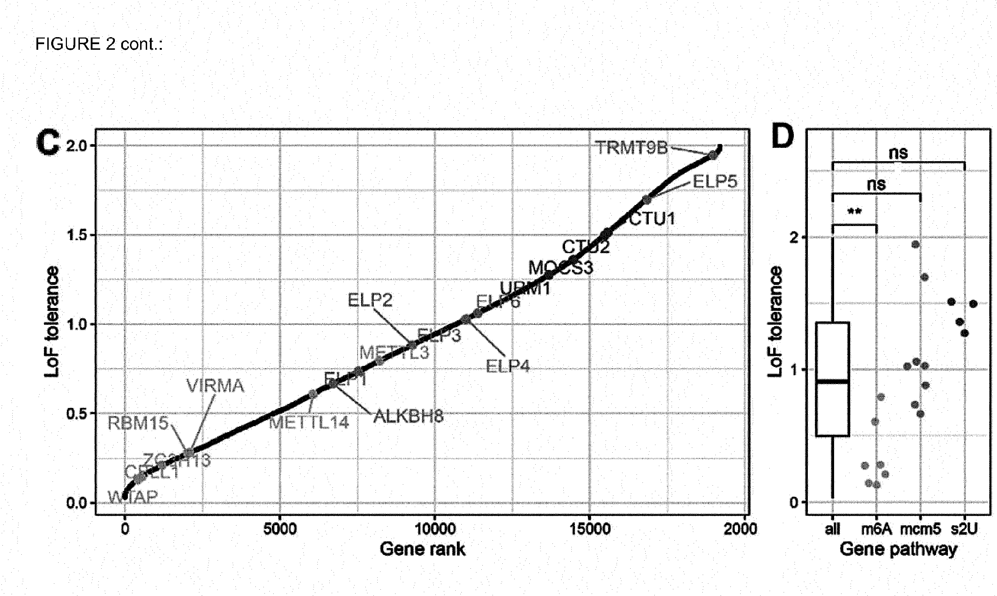

Figure 2 shows A. Co-occurrence of somatic mutations in m6A and mcm5s2U biogenesis factors. Heatmaps of pairwise loss of function (LoF) mutation patterns indicating co-occurring and mutual exclusive mutations for 24 cancer types with mutations in at least one biogenesis pathway component. Significant pairs are marked by dots (p-Value < 0.01; Fisher's exact test). Biogenesis factors for m6A are highlighted by the number (1), biogenesis factors for carboxymethylation (mcm⁵U) are highlighted by the number (2), and biogenesis factors for thiolation (mcm5s2U) are highlighted by the number (3). The area of cross-pathway co-occurrence between m6A and mcm5s2U biogenesis is indicated by a dashed box. B-D: m6A biogenesis is less tolerant to germline mutations than mcm5s2U biogenesis. B. Observed vs. expected ratios (±95% confidence interval) of germline loss-of-function (LoF) variants for m6A and mcm5s2U biogenesis factors (data from gnomAD). C. Ranking genes by germline LoF tolerance separates m6A and mcm5s2U biogenesis factors. D. Germline LoF tolerance of the m6A biogenesis pathway is significantly lower than the genome average, whereas mcm5 and s2U pathways are not significantly different. (Wilcoxon test; **: p ≤ 0.01).

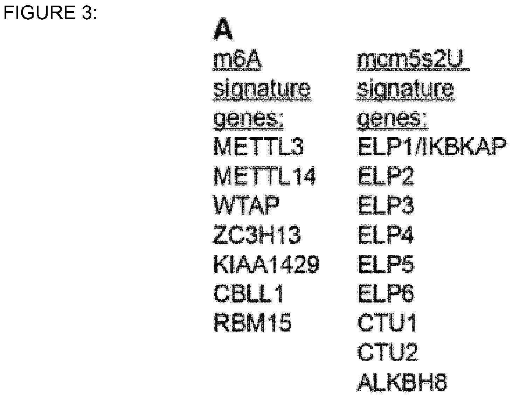

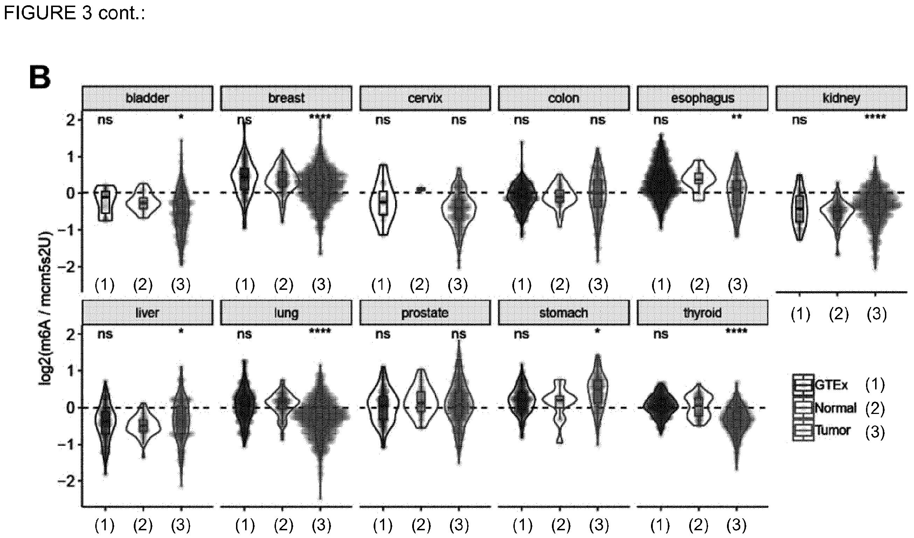

Figure 3 shows m6A and mcm5s2U biogenesis signatures differ in normal tissues and tumors. A. Genes involved in the biogenesis of m6A and mcm5s2U that are used to calculate the expression signatures. B. m6A/mcm5s2U signature expression balance differs between tissues and tumors. Normalized, batch corrected expression data was used. "GTEx": Normal tissue from GTEx; "Normal": Tumor-adjacent normal tissue from TCGA; "Tumor": Tumor tissue from TCGA.

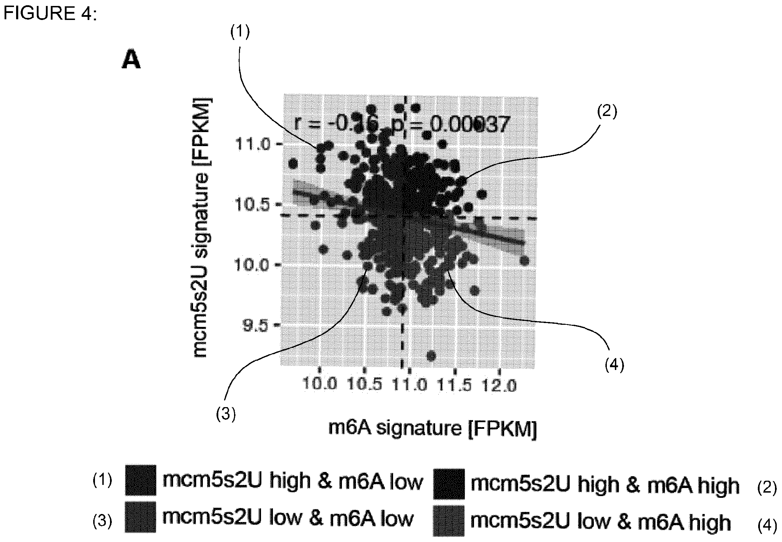

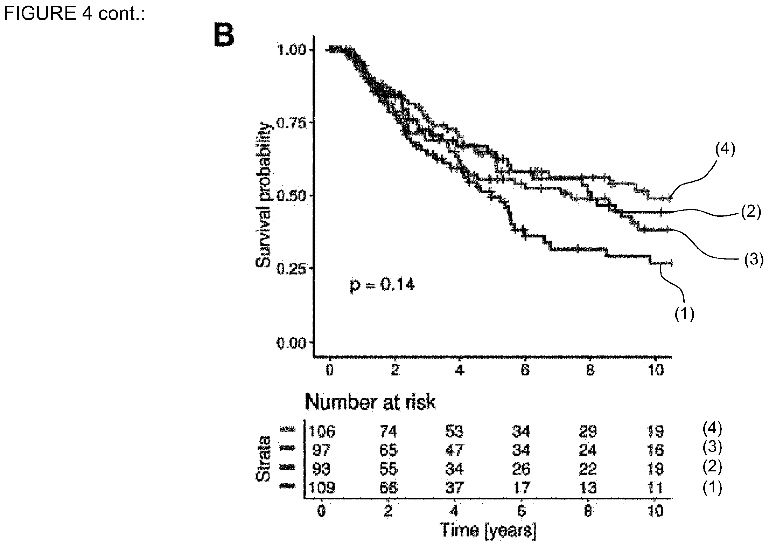

Figure 4 shows m6A and mcm5s2U expression signatures predict melanoma survival. A. Scatterplot of m6A and mcm5s2U expression signature in the TCGA melanoma cohort (SKCM). The numbers (1), (2), (3), and (4) indicate stratification by signature expression, the numbers (2) and (3) are tumors with balanced m6A/mcm5s2U. B. Kaplan-Meier survival analysis of the TCGA melanoma cohort. Tumors were stratified as in A. Hazard ratio for high mcm5s2U vs. high m6A is 1.44 (95% CI: 0.98-2.13).

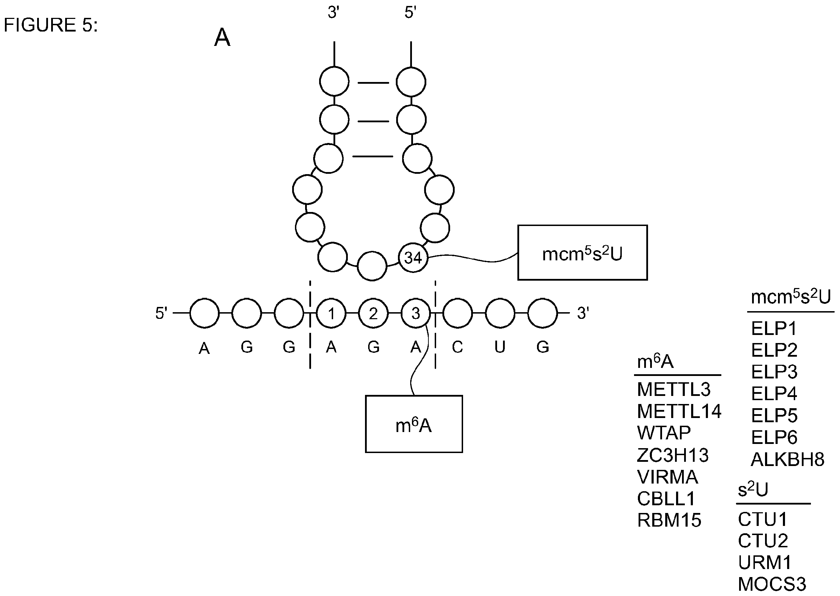

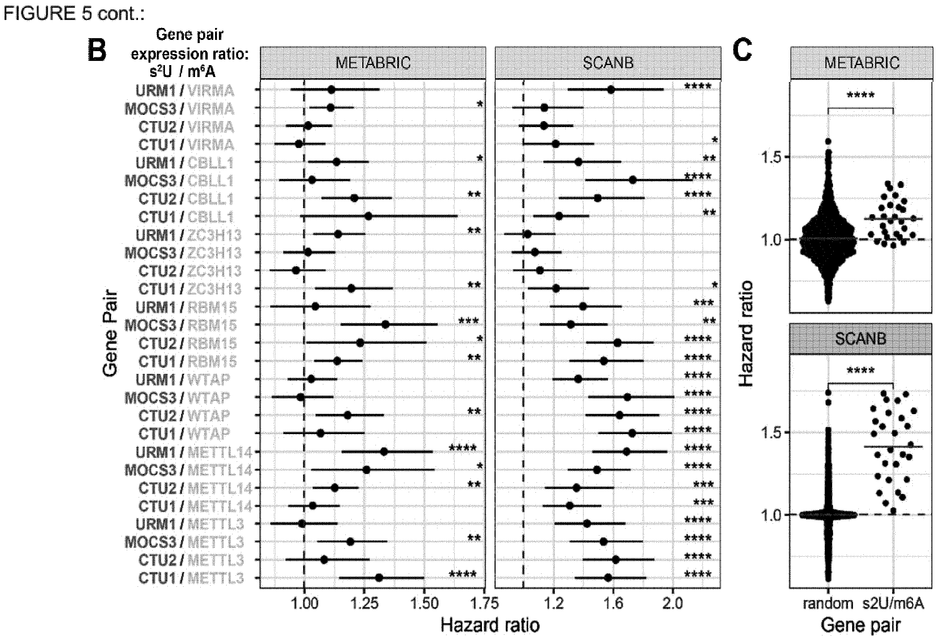

Figure 5 shows shifted expression balance towards mcm5s2U correlates with an increased hazard ratio for most individual pairs of mcm5s2U and m6A biogenesis factors. A. Upper panel: Schematic of the m6A-modified mRNA codon and the mcm5s2U-modified tRNA anticodon. Lower panel: List of biogenesis factors for m6A and mcm5s2U carboxymethylation (mcm⁵U) and thiolation (mcm5s2U). B. Univariable Cox proportional hazards for mcm5s2U ("s²U") and m6A biogenesis factor gene pair expression ratios. For each pair, the expression ratio was calculated and used to predict overall survival in the METABRIC and SCANB breast cancer cohorts (hazard ratio ± 95% confidence interval). C. In both cohorts, hazard ratios of mcm5s2U ("s²U")/m6A gene pairs are significantly higher than those of 1000 randomly selected gene pairs. (****: p ≤ 0.0001; Wilcoxon test)

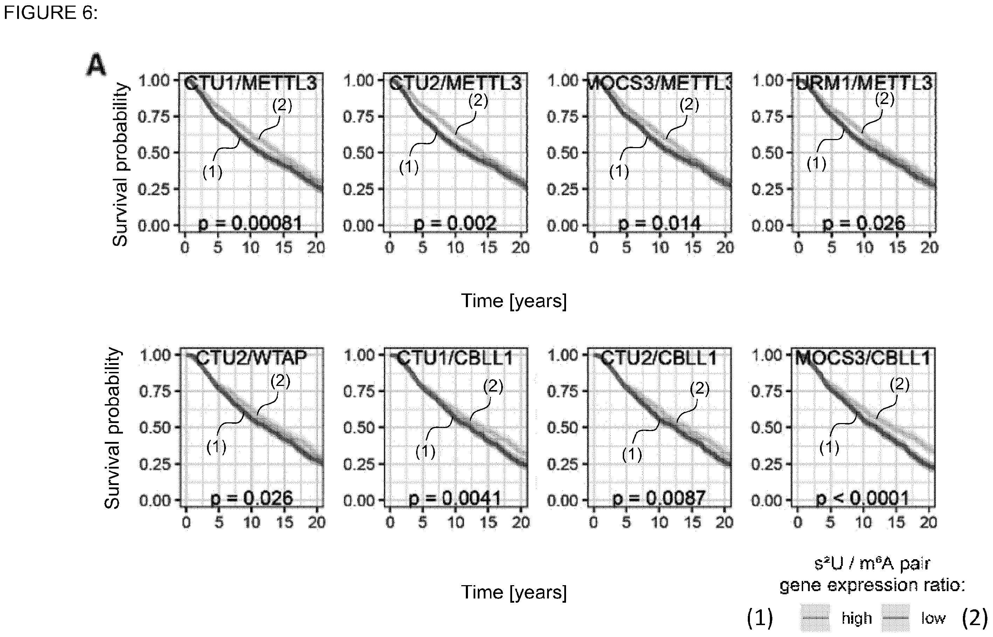

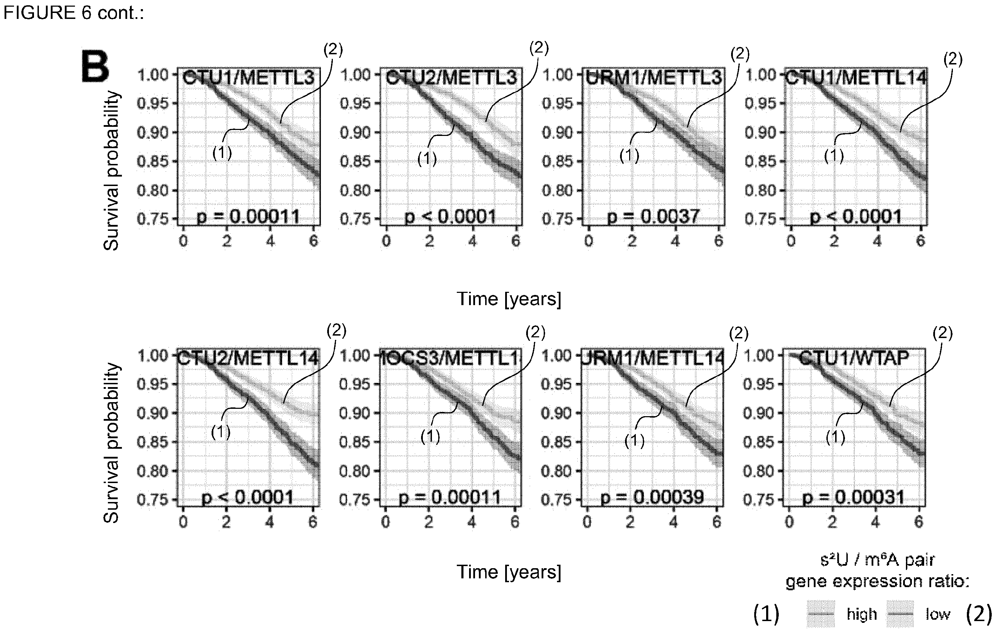

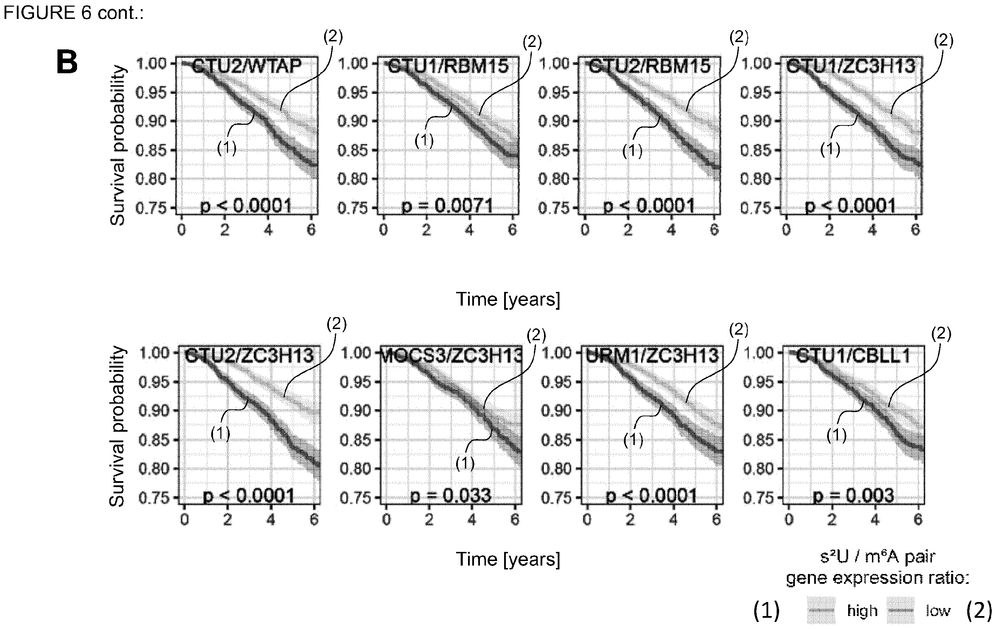

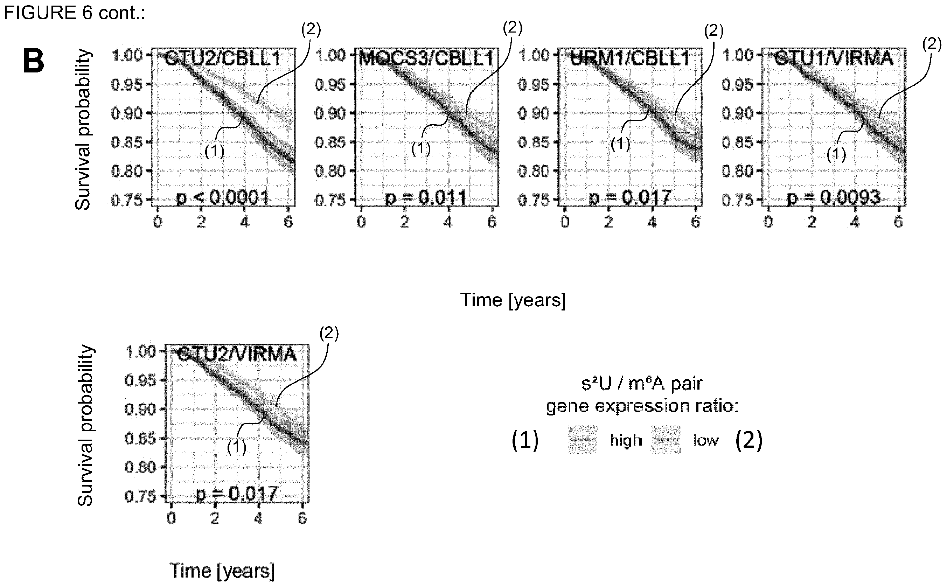

Figure 6 shows Kaplan-Meier survival curves confirm the poor overall survival of patients with a shifted gene pair balance towards mcm5s2U biogenesis factors. A. Kaplan-Meier curves for all significant gene pairs in the METABRIC cohort. Expression ratios of mcm5s2U ("s²U")/m6A biogenesis factor gene pairs were categorized into high (more mcm5s2U; referenced by number (1)) and low (more m6A; referenced by number (2)). 95% confidence intervals and p-values of logrank-test are indicated. B. Same as in (A), but for the SCANB cohort.
Figure 7 shows that the combined m⁶A and mcm⁵s²U signature predicts specific cancer drug vulnerabilities. A. Gene set enrichment analysis of EGFR signaling genes in the genome-wide dependency correlations with the m⁶A/mcm⁵s²U signature. B. Correlations between the m6A/mcm⁵s²U signature and CRISPR gene dependencies. EGFR pathway components are labelled. C. Correlations between the m6A/mcm⁵s²U signature and drug vulnerabilities (EGFR inhibitors are labeled). D and E. m6A/mcm⁵s²U plotted against drug vulnerabilities for gefitinib (D) and lapatinib (E) in cell lines negative or positive for EGFR expression.

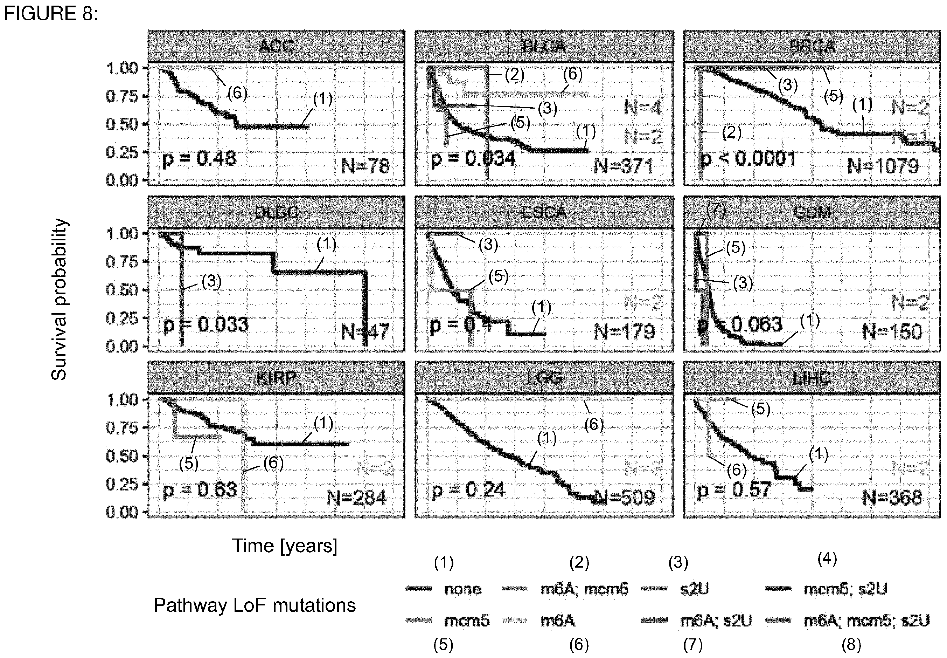

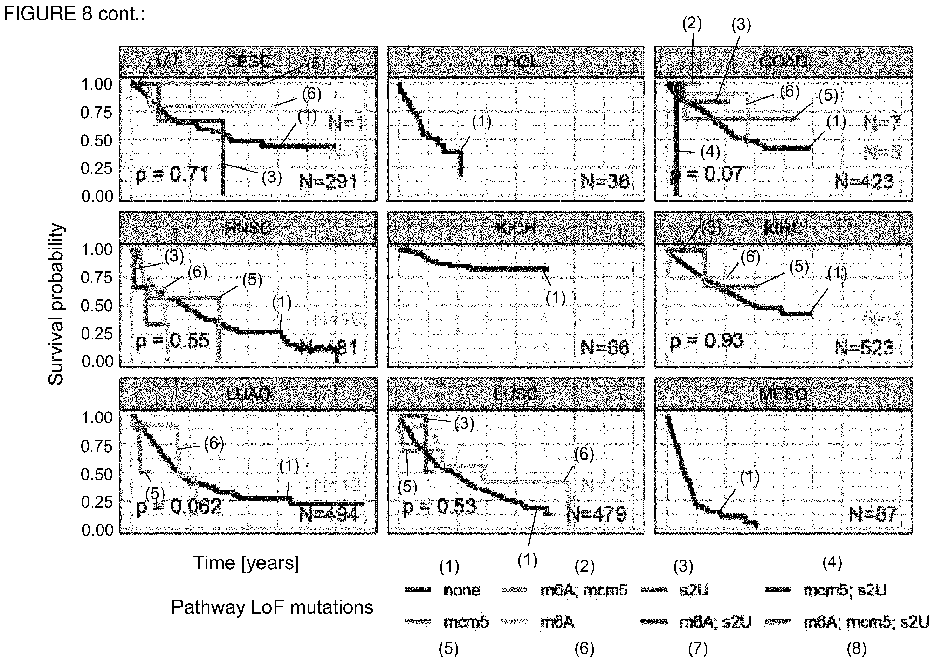

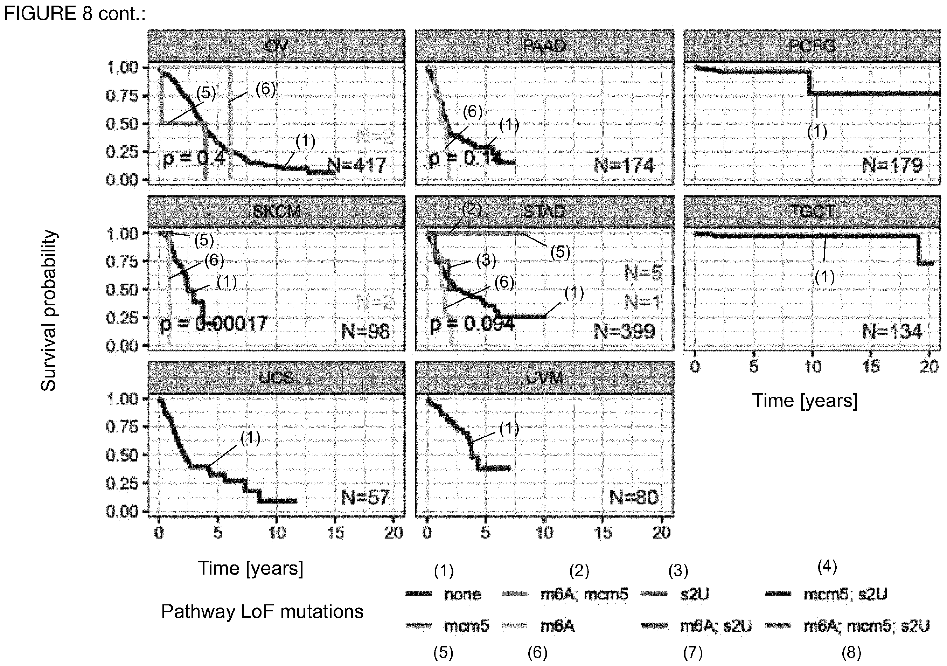

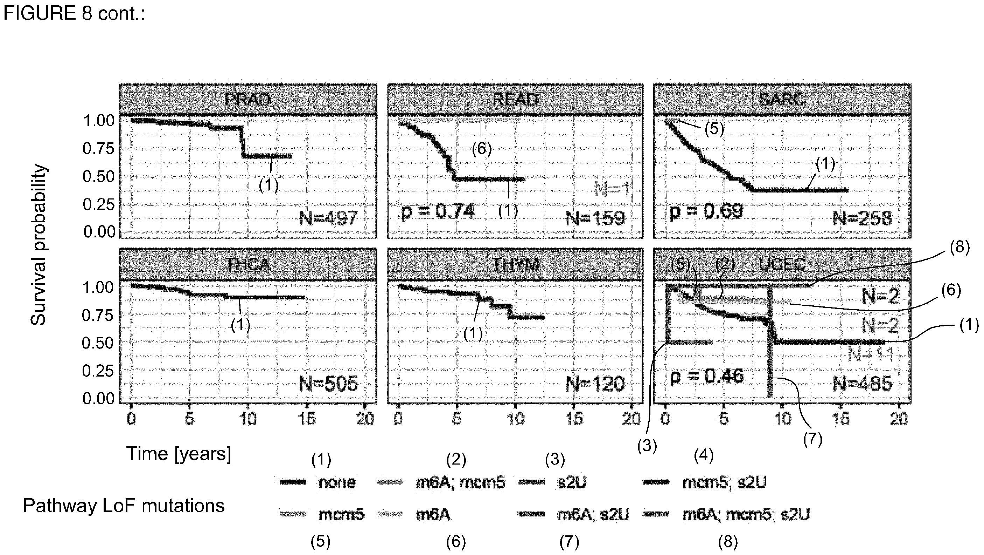

Figure 8 shows that tumor somatic mutations in m6A and mcm5s2U biogenesis pathways are not associated with significantly different overall survival. For each combination of loss of function (LoF) mutations in m6A/mcm5s2U ("s²U") biogenesis pathway components, overall survival in TCGA cancer cohorts was analyzed.

The sequences of SEQ ID Nos: 1 to 18 show various possible DRACH-motifs:
SEQ ID No. 1: AGACU
SEQ ID No. 2: GGACU
SEQ ID No. 3: UGACU
SEQ ID No. 4: AAACU
SEQ ID No. 5: GAACU
SEQ ID No. 6: UAACU
SEQ ID No. 7: AGACA
SEQ ID No. 8: GGACA
SEQ ID No. 9: UGACA
SEQ ID No. 10: AAACA
SEQ ID No. 11: GAACA
SEQ ID No. 12: UAACA
SEQ ID No. 13: AGACC
SEQ ID No. 14: GGACC
SEQ ID No. 15: UGACC
SEQ ID No. 16 AAACC
SEQ ID No. 17: GAACC
SEQ ID No. 18: UAACC
SEQ ID No. 19: AGGAGACUG

### EXAMPLES

Certain aspects and embodiments of the invention will now be illustrated by way of example and with reference to the description, figures and a table set out herein. Such examples of the methods, uses and other aspects of the present invention are representative only, and should not be taken to limit the scope of the present invention to only such representative examples.

The examples show:

### Example 1: tRNA anticodon modifications decode m6A-modified codons

To understand the functions of m6A epitranscriptomic gene regulation, the inventors used transcriptional shutoff datasets, and calculated decay rates by linear regression of log-transformed expression data over time. Where available, the inventors used preprocessed expression values (table 1). Otherwise, raw sequencing data was used to determine expression by alignment and read counting or microarray data was obtained from GEO (see table 1). In case preprocessed data contained more than one measurement per gene (i.e. different isoforms of probes), the gene average was used. Because only relative decay rates were analyzed, spike-in normalization was omitted. For SLAM-Seq data analysis, published conversion rates (see table 1) were averaged per gene and decay rates. Stabilization upon METTL3 KO or emetine treatment was determined by subtracting WT from KO decay rates or control from emetine decay rates, respectively.

**Table 1: Links to datasets and accession numbers used in this study.**

| **Dataset** | **Accession number(s)** | **File link(s)** |
|---|---|---|
| LAIC | | https://media.nature.com/original/nature-assets/nmeth/journal/v13/n8/extref/nmeth.389 8-S2.xlsx |
| DRUID | | ftp://ftp.ncbi.nlm.nih.gov/geo/series/GSE99nnn /GSE99517/suppl/GSE99517%5FHEK%2E4SU% 2EHL%2EDRUID%2Eone%2Ecsv%2Egz |
| Ke METTL3 KO mESC decay | | https://www.ncbi.nlm.nih.gov/geo/download/?a cc=GSE86336&format=file |
| Schwartz m6A-IP HEK293 | IP = SRR1182595, input = SRR1182596 | |
| Geula m6A-IP | IP = SRR1596085, input = SRR1596086 | |
| Geula METTL3 KO mESC decay | | ftp://ftp.ncbi.nlm.nih.gov/geo/series/GSE61nnn/ GSE61994/suppl/GSE61994%5FDGE%5Fnormali zed%5Flevels%5FRefSeq%2Etxt%2Egz |
| Herzog METTL3 KO mESC decay | | https://www.ncbi.nlm.nih.gov/geo/download/?a cc=GSE99974&format=file |
| Riboseq | SRR2433794 | |
| Riboseq | SRR2075925 | |
| Arsenite polysome gradients | | ftp://ftp.ncbi.nlm.nih.gov/geo/series/GSE44nnn /GSE44379/suppl/GSE44379%5Fars%5Fnoars% 5Fpolysome%5Fprofiles%2Etxt%2Egz |
| Arsenite ribosome profiling | | ftp://ftp.ncbi.nlm.nih.gov/geo/series/GSE55nnn /GSE55195/suppl/GSE55195%5FSupplementalTa ble1%2Exlsx%2Egz |

The inventors focused on analyzing the third position of codons, because m6A sites at the third codon position are more conserved between mouse and human. This position also has the highest DRACH methylation rate, and ribosome pauses are strongest. The third position of a codon is further special, as it tolerates non-Watson/Crick ("wobble") base pairing with the tRNA anticodon to enable the degeneracy of the genetic code. Intriguingly, modified nucleotides in the tRNA anticodon often modulate wobble decoding and can influence tRNA selection.

A tRNA anticodon nucleotide modification that juxtaposes adenosines at the wobble position is mcm5s2U. tRNAs that are modified by mcm5s2U decode codons AAA, CAA and GAA in yeast and humans. Recently, it was shown that in humans AGA is also decoded by an mcm5s2U-modified tRNA. The inventors hypothesized that mcm5s2U could be involved in decoding m6A-modified AAA, GAA and AGA codons. To test whether mcm5s2U is involved in decoding m6A, the inventors generated HEK293 cell lines in which IKBKAP or CTU2 were knocked out using CRISPR to efficiently deplete mcm5s2U.

The inventors then used ribosome profiling to determine A-site occupancies, classifying DRACH codons into m6A-modified and unmodified codons. Raw sequencing data was trimmed for the appropriate adapter with flexbar and aligned with STAR. Reads with a length of 28, 29 or 30 nt were used. Offsets of RPF 5'-ends to A-site codons were determined by analyzing RPF 5'-end coverage at start and stop codons and were found to be 15 nt for all datasets analyzed. Normalized A-site coverages were calculated for all nucleotides in the transcriptome. For this, a z-score normalization of the raw counts was performed in a window of -60 nt to +60 nt surrounding the position of interest. For codons, the normalized A-site coverage at position 1 (i.e. of ribosomes translating it in-frame) was used. For window coverage plots, the average z-scores at each position are plotted.

Raw sequencing data was combined, and the full dataset was aligned with STAR and deduplicated with UMI-tools. Alignment was performed in end-to-end mode to assess intra-read distribution of C to T transitions, but the first two positions were excluded for m6A site calling. Transitions were determined with Rsamtools pileup and m6A-sites were called by requiring at least 2 transitions and a transition rate between 4 % and 40 %. For each m6A-modified codon, the inventors selected the nearest unmethylated codon of the same type in the same CDS. This was done separately for unmethylated codons that either are in a DRACH motif or not. The inventors did not exclude m6A sites for which no matching codon in the same CDS could be found, but the fraction of these sites is small.

Upon analyzing ELP1/IKBKAP and CTU2 knockout cells, the inventors found that A-site occupancies did not change for most codons in IKBKAP and CTU2 knockout cells (Fig. 1B). The most striking observation was, however, an exceptionally high A-site occupancy of m6A-modified versions of codons AGA and GAA in IKBKAP and CTU2 knockout cells (Fig. 1B). This indicated that ribosomes pause when these methylated codons are in their A-site and mcm5s2U-modified cognate tRNAs are not available. Accordingly, the inventors reasoned that a function of mcm5s2U is to facilitate decoding of m6A-modified codons.

Thus, the inventors discovered a mechanism by which m6A connects nuclear mRNA processing to tRNA decoding. The inventors found that the amount of m6A that is deposited in the protein coding sequence (CDS) of an mRNA correlates with the length of the exons in this region, and CDS m6A leads to the slow decoding of a subset of modified codons in *vivo.* However, when m6A is at the wobble position, decoding can be facilitated by the tRNA anticodon modification 5-methoxycarbonylmethyl-2-thiouridine (mcm5s2U), thereby revealing an unanticipated link between the mRNA and tRNA epitranscriptomes. The m6A-mcm5s2U-system controls mRNA half-life in a translation-dependent manner and thus connects the exon-intron architecture of mRNAs to their turnover.

### Example 2: Co-occurrence of somatic mutations in m6A and mcm5s2U biogenesis factors

The inventors analyzed 24 cancer types with mutations in at least one of the biogenesis pathway components of the m6A biogenesis pathway, the carboxymethylation (mcm⁵U) biogenesis pathway, and the thiolation (mcm5s2U) biogenesis pathway (Fig. 2). The inventors further analyzed cross-pathway co-occurrence of mutations between m6A and mcm5s2U biogenesis factors in cancer type-specific plots and found a significant enrichment of mutations co-occurring between m6A and mcm5s2U pathway components (Fig. 2), i.e. tumors with mutations in the m6A pathway tend to also have mutations in the mcm5s2U pathway and vice versa. This suggests that m6A and mcm5s2U are key players in cancer. The inventors further compared somatic mutations to germline variants in components of the m6A biogenesis pathway, of the carboxymethylation (mcm⁵U) biogenesis pathway, and of the thiolation (mcm5s2U) biogenesis pathway (Fig. 2), and found mcm5s2U is more tolerant to germline loss of function mutations than m6A. This might have an impact on cancer predisposition.

### Example 3: m6A and mcm5s2U biogenesis signatures differ in normal tissues and tumors

The inventors used genes involved in the biogenesis of m6A and mcm5s2U to calculate expression signatures of m6A and mcm5s2U (Fig. 3A). A ratio of m6A/mcm5s2U was then used to analyze whether the m6A/mcm5s2U signature gene expression is balanced. Interestingly, the m6A/mcm5s2U signature expression balance differs between normal tissues, and tumors (Fig. 3B).

The inventors found that the m6A/mcm5s2U signature gene expression ratio can be used to group diseases, conditions or disorders into the following groups:
**(a)** a disease, condition or disorder associated with a higher level of m6A-modified mRNA and a lower level of mcm5s2U-modified tRNA compared to a control,
**(b)** a disease, condition or disorder associated with a higher level of m6A-modified mRNA and an unchanged/normal level of mcm5s2U-modified tRNA compared to a control,
**(c)** a disease, condition or disorder associated with a higher level of m6A-modified mRNA and a higher level of mcm5s2U-modified tRNA compared to a control,
**(d)** a disease, condition or disorder associated with a lower level of m6A-modified mRNA and a higher level of mcm5s2U-modified tRNA compared to a control,
**(e)** a disease, condition or disorder associated with a lower level of m6A-modified mRNA and a unchanged/normal level of mcm5s2U-modified tRNA compared to a control,
**(f)** a disease, condition or disorder associated with a lower level of m6A-modified mRNA and a lower level of mcm5s2U-modified tRNA compared to a control,
**(g)** a disease, condition or disorder associated with a unchanged/normal level of m6A-modified mRNA and a lower level of mcm5s2U-modified tRNA compared to a control, and
**(h)** a disease, condition or disorder associated with a unchanged/normal level of m6A-modified mRNA and a higher level of mcm5s2U-modified tRNA compared to a control.

The control is preferably a cohort-intrinsic reference sample. The inventors found that if the m6A/mcm5s2U signature gene expression ratio is such that the result is indicative for:
- a disease, condition or disorder associated with a lower level of m6A-modified mRNA and a higher level of mcm5s2U-modified tRNA (see **(d)** above),
- a disease, condition or disorder associated with a lower level of m6A-modified mRNA and a unchanged/normal level of mcm5s2U-modified tRNA (see **(e)** above), or
- a disease, condition or disorder associated with a unchanged/normal level of m6A-modified mRNA and a higher level of mcm5s2U-modified tRNA (see **(h)** above), the disease condition or disorder is a cancer disease, which is preferably selected from bladder cancer, breast cancer, esophagus cancer, lung cancer, and thyroid cancer.

Therefore, a particularly preferred embodiment of this invention relates to the use of an mcm5S2U inhibitor in the treatment and/or prevention of bladder cancer, breast cancer, esophagus cancer, lung cancer, and thyroid cancer. Most preferably, said cancer to be treated is breast cancer.

The inventors further found that if the m6A/mcm5s2U signature gene expression ratio is such that the result is indicative for
- a disease, condition or disorder associated with a higher level of m6A-modified mRNA and a lower level of mcm5s2U-modified tRNA (see **(a)** above),
- a disease, condition or disorder associated with a higher level of m6A-modified mRNA and a unchanged/normal level of mcm5s2U-modified tRNA (see **(b)** above), or
- a disease, condition or disorder associated with a unchanged/normal level of m6A-modified mRNA and a lower level of mcm5s2U-modified tRNA (see **(g)** above), the disease condition or disorder is a cancer disease that is preferably selected from kidney cancer, liver cancer, and stomach cancer.

Thus, the inventors discovered that cells balance the expression of m6A and mcm5s2U biogenesis machineries and failure to do so is associated with particular diseases, such as cancer, suggesting that the m6A/mcm5s2U axis has evolved as an epitranscriptomic mechanism to regulate gene expression and control cell differentiation. In healthy human tissues, the expression of biogenesis factors for m6A and mcm5s2U is highly correlated. Most importantly, CRISPR gene-dependency data shows that cancer cells that depend on m6A biogenesis factors also require mcm5s2U biogenesis factors and vice-versa. These results suggest that the m6A- and mcm5s2U-epitranscriptomes are intricately balanced; thus, modification of the tRNA anticodon with mcm5s2U is counteracting an imbalance of m6A, and vice versa.

### Example 4: m6A and mcm5s2U expression signatures predict survival in melanoma and breast cancer cohorts

A scatterplot of m6A and mcm5s2U expression signature in the TCGA melanoma cohort (SKCM) shows, that m6A and mcm5s2U expression signatures predict melanoma survival (Fig. 4). The inventors found that if the m6A/mcm5s2U signature gene expression ratio is such that the result is indicative for a lower level of m6A-modified mRNA and a higher level of mcm5s2U-modified tRNA, the prognosis for said subject is lower than for a subject suffering from the same disease, condition or disorder and not showing a lower level of m6A-modified mRNA and a higher level of mcm5s2U-modified tRNA. Furthermore, the inventors found that if the m6A/mcm5s2U signature gene expression ratio is such that the result is indicative for a higher level of m6A-modified mRNA and a lower level of mcm5s2U-modified tRNA, the prognosis for said subject is higher than for a subject suffering from the same disease, condition or disorder and not showing such result.

The inventors further used different mcm5s2U ("s2U")/m6A biogenesis factor gene pair expression ratios to predict overall survival in the METABRIC and SCANB breast cancer cohorts (Fig. 5). In both cohorts, hazard ratios of mcm5s2U ("s²U")/m6A gene pairs are significantly higher than those of 1000 randomly selected gene pairs (Fig. 5C). Then, the inventors used Kaplan-Meier survival curves to confirm the poor overall survival of patients with a shifted gene pair balance towards mcm5s2U biogenesis factors in the METABRIC cohort (Fig. 6A) and SCANB cohort (Fig. 6B). The expression ratios of mcm5s2U ("s²U")/m6A biogenesis factor gene pairs were categorized into high (more mcm5s2U) and low (more m6A).

### Example 5: A combined m⁶A and mcm⁵s²U signature predicts specific cancer cell line vulnerabilities

The inventors used the combined m⁶A/mcm⁵s²U signature to probe cancer cell line vulnerabilities. Analysis of genome-wide CRISPRi gene dependencies revealed an enrichment of EGFR signaling pathway components in cell lines with a high m⁶A/mcm⁵s²U signature (Fig. 7A). In line with this, EGFR is one of the top gene dependencies that correlate with a high m⁶A/mcm⁵s²U signature (Fig. 7B). Receptor tyrosine kinases of the EGFR/ErbB family are frequently overexpressed in tumors and targeted by several FDA-approved tyrosine kinase inhibitors (TKIs) including lapatinib and gefitinib. The inventors observed that sensitivity to these EGFR-targeting TKIs is correlated with the m⁶A/mcm⁵s²U signature (Fig. 7C). A detailed analysis of these drug vulnerabilities revealed that a high m⁶A/mcm⁵s²U signature correlates with increased sensitivity to lapatinib and gefitinib in EGFR-positive cells. Cell lines in the lowest signature quartile are as insensitive to EGFR inhibitors as EGFR-negative cells. Thus, the combined m6A/mcm5s2U signature serves as a biomarker for the sensitivity of cancer cells to EGFR inhibition.

### Example 6: Tumor somatic mutations in m6A and mcm5s2U biogenesis pathways are not associated with significantly different overall survival

The inventors analyzed whether tumor somatic mutations in m6A and mcm5s2U biogenesis pathways are associated with different overall survival in TCGA cancer cohorts. However, the results show that tumor somatic mutations in m6A and mcm5s2U biogenesis pathways are not associated with significantly different overall survival in general (Fig. 8). While for some cancers, significant differences in survival (e.g. breast cancer [BRCA]) are detected, this is based on very few mutant cases. Thus, further confirming an imbalance of mcm5s2U to m6A ratio to be the main trigger for a different overall survival in TCGA cancer cohorts.

### REFERENCES

The references are:
1. Geula, S., Moshitch-Moshkovitz, S., Dominissini, D., Mansour, A.A., Kol, N., Salmon-Divon, M., Hershkovitz, V., Peer, E., Mor, N., Manor, Y.S., et al. (2015). m6A mRNA methylation facilitates resolution of naive pluripotency toward differentiation. Science 347, 1002-1006.
2. Aguilo, F., Zhang, F., Sancho, A., Fidalgo, M., Di Cecilia, S., Vashisht, A., Lee, D.-F., Chen, C.-H., Rengasamy, M., Andino, B., et al. Coordination of m6A mRNA Methylation and Gene Transcription by ZFP217 Regulates Pluripotency and Reprogramming. Cell Stem Cell o. Available at: http://www.cell.com/article/S1934590915004129/abstract [Accessed November 6, 2015].
3. Yoo, H., Son, D., Jang, Y.-J., and Hong, K. (2016). Indispensable role for mouse ELP3 in embryonic stem cell maintenance and early development. Biochemical and Biophysical Research Communications 478, 631-636.
4. Qi, L., Zhang, X., Zhai, H., Liu, J., Wu, F., Li, C., and Chen, Q. (2019). Elongator Is Required for Root Stem Cell Maintenance by Regulating SHORTROOT Transcription. Plant Physiology 179, 220-232.
5. Delaunay, S., and Frye, M. (2019). RNA modifications regulating cell fate in cancer. Nature Cell Biology 21, 552-559.
6. Delaunay, S., Rapino, F., Tharun, L., Zhou, Z., Heukamp, L., Termathe, M., Shostak, K., Klevernic, I., Florin, A., Desmecht, H., et al. (2016). Elp3 links tRNA modification to IRES-dependent translation of LEF1 to sustain metastasis in breast cancer. Journal of Experimental Medicine 213, 2503-2523.
7. Fry, N.J., Law, B.A., Ilkayeva, O.R., Carraway, K.R., Holley, C.L., and Mansfield, K.D. (2018). N6-methyladenosine contributes to cellular phenotype in a genetically-defined model of breast cancer progression. Oncotarget 9, 31231-31243.
8. Goodarzi, H., Nguyen, H.C.B., Zhang, S., Dill, B.D., Molina, H., and Tavazoie, S.F. (2016). Modulated Expression of Specific tRNAs Drives Gene Expression and Cancer Progression. Cell 165, 1416-1427.

## Claims

1. Use of a biomarker consisting of a N6-methyladenosine (m6A)-mRNA modification and a 5-methoxycarbonylmethyl-2-thiouridine (mcm5s2U)-tRNA anticodon modification in an *in-vitro* method for the prognosis, diagnosis, stratification and/or monitoring of a disease, condition or disorder in a subject.

2. The use according to claim 1, wherein the method comprises quantifying the amount of the m6A-mRNA modification and mcm5s2U-tRNA anticodon modification directly via assessing the modified RNA nucleotide.

3. The use according to claim 2, wherein the method comprises quantifying the amount of the m6A-mRNA modification and mcm5s2U-tRNA anticodon modification directly via assessing the modified RNA nucleotide via mass spectrometry.

4. The use according to any one of claims 1 to 3, wherein the method comprises the determination of the level of at least one m6A-mRNA modification biomarker and the level, mutation status, and/or activity of at least one mcm5s2U-tRNA anticodon modification biomarker as a combined biomarker, wherein the m6A-mRNA modification biomarker is METTL3, METTL14, WTAP, VIRMA, RBM15, ZC3H13, YTHDF1, YTHDF2, YTHDF3, FTO, KIAA1429, CBLL1, RBM15, and/or ALKBH5; and the mcm5s2U-tRNA anticodon modification biomarker is selected from ELP1/IKBKAP, ELP2, ELP3, ELP4, ELP5, ELP6, CTU1, CTU2, MOCS3, URM1, and/or ALKBH8.

5. The use according to claim 4, wherein said at least one mcm5s2U-tRNA anticodon modification biomarker comprises a panel of CTU1, CTU2, MOCS3, and URM1, and/or wherein said at least one mcm5s2U-tRNA anticodon modification biomarker consists of a panel of CTU1, CTU2, MOCS3, and URM1.

6. The use according to any one of claims 1 to 5, wherein the method is for the prognosis, diagnosis, and/or monitoring of a disease, condition or disorder in a subject, comprising the steps of
**(a)** Providing an obtained biological sample of said subject,
**(b)** Determining the level, mutation status, and/or activity of at least one m6A-mRNA modification biomarker indicative and/or selective for the status and/or activity of the pathway regulating m6A mRNA modification in a cell in said biological sample, by quantifying the amount of the m6A modification directly via assessing the modified RNA nucleotide and/or by quantifying the presence of at least one m6A-mRNA modification biomarker selected from METTL3, METTL14, WTAP, VIRMA, RBM15, ZC3H13, YTHDF1, YTHDF2, YTHDF3, FTO, KIAA1429, CBLL1, RBM15, and/or ALKBH5,
**(c)** Determining the level, mutation status, and/or activity of at least one mcm5s2U-tRNA anticodon modification biomarker indicative and/or selective for the status and/or activity of the pathway regulating mcm5s2U-tRNA anticodon modification in a cell in said biological sample, by quantifying the amount of the mcm5s2U-tRNA anticodon modification directly via assessing the modified RNA nucleotide and/or by quantifying the presence of at least one mcm5s2U-tRNA anticodon modification biomarker selected from ELP1/IKBKAP, ELP2, ELP3, ELP4, ELP5, ELP6, CTU1, CTU2, MOCS3, URM1, and/or ALKBH8,
**(d)** Comparing the determined level, mutation status, and/or activity of said m6A-mRNA modification biomarker and said mcm5s2U-tRNA anticodon modification biomarker in steps **(b)** and **(c)** with a reference sample or a reference value,
wherein a differential level, mutation status, and/or activity of said m6A-mRNA modification biomarker and/or mcm5s2U-tRNA anticodon modification biomarker in the biological sample from said subject compared to said reference sample or reference value is indicative for a reduced time of progression free survival (PFS) and/or overall survival (OS) of a patient suffering from a disease, condition or disorder, or wherein a differential level, mutation status, and/or activity of said m6A-mRNA modification biomarker and/or mcm5s2U-tRNA anticodon modification biomarker in the biological sample from said subject compared to said reference sample or reference value is indicative for the presence of said disease, condition or disorder in said subject.

7. The use according to any one of claims 1 to 5, wherein the method is for stratifying a patient for a treatment, said method comprising, as a first step, identifying a differential level, mutation status, and/or activity of at least one m6A-mRNA modification biomarker and/or at least one mcm5s2U-tRNA anticodon modification biomarker in the biological sample from said patient compared to a reference sample or reference value, and, as a second step, deciding in favor of or against said treatment based on said differential level, mutation status, and/or activity, wherein said differential level, mutation status, and/or activity is identified by using a method comprising the steps of:
**(a)** Providing an obtained biological sample of said patient,
**(b)** Determining the level, mutation status, and/or activity of at least one m6A-mRNA modification biomarker indicative and selective for the status and/or activity of the pathway regulating m6A-mRNA modification in a cell in said biological sample, by quantifying the amount of the m6A modification directly via assessing the modified RNA nucleotide and/or by quantifying the presence of at least one m6A-mRNA modification biomarker selected from METTL3, METTL14, WTAP, VIRMA, RBM15, ZC3H13, YTHDF1, YTHDF2, YTHDF3, FTO, KIAA1429, CBLL1, RBM15, and/or ALKBH5,
**(c)** Determining the level, mutation status, and/or activity of at least one mcm5s2U-tRNA anticodon modification biomarker indicative and selective for the status and/or activity of the pathway regulating mcm5s2U-tRNA anticodon modification in a cell in said biological sample, by quantifying the amount of the mcm5s2U-tRNA anticodon modification directly via assessing the modified RNA nucleotide and/or by quantifying the presence of at least one mcm5s2U-tRNA anticodon modification biomarker selected from ELP1/IKBKAP, ELP2, ELP3, ELP4, ELP5, ELP6, CTU1, CTU2, MOCS3, URM1, and/or ALKBH8, and
**(d)** Comparing the determined level, mutation status, and/or activity of said m6A-mRNA modification biomarker and mcm5s2U-tRNA anticodon modification biomarker in steps **(b)** and **(c)** with a reference sample or a reference value;
preferably, wherein said treatment is a treatment targeting an m6A-mRNA modification in a cell and/or an mcm5s2U-tRNA anticodon modification in a cell, preferably wherein said treatment inhibits or activates the m6A-MTC, an m6A reader protein, an m6A eraser protein, and/or the elongator complex.

8. The use according to claim 7, wherein based on the differential level, mutation status, and/or activity of said m6A-mRNA modification biomarker and/or mcm5s2U-tRNA anticodon modification biomarker in the biological sample from said subject compared to said reference sample or value in step **(d),** it is decided in favor of enhancing or inhibiting the level, mutation status, and/or activity of the pathway regulating m6A-mRNA modification, and/or enhancing or inhibiting the level, mutation status, and/or activity of the pathway regulating mcm5s2U-tRNA anticodon modification.

9. The use according to any one of claims 1 to 8, wherein said disease, condition or disorder is cancer, and/or a disease that shows a differential level, mutation status, and/or activity of the pathway regulating m6A-mRNA modification in a cell, and/or that shows a differential level, mutation status, and/or activity of the pathway regulating mcm5s2U-tRNA anticodon modification in a cell, preferably wherein said cancer is selected from breast cancer, melanoma, glioblastoma, endometrial cancer, Acute Myeloid Leukemia (AML), lung cancer, bladder cancer, hepatocellular cancer, medulloblastoma, kidney cancer, liver cancer, head and neck cancer, prostate cancer, pancreatic cancer, stomach cancer, colon cancer, thyroid cancer, esophageal cancer, brain cancer, colorectal cancer, gastric cancer, cervical cancer, ovarian cancer, cancer of the urinary tract, renal cancer, carcinoma, and a tumor that shows a differential level, mutation status, and/or activity of the pathway regulating m6A-mRNA modification in a cell, and/or regulating mcm5s2U-tRNA anticodon modification in a cell, more preferably wherein said cancer is breast cancer.

10. The use according to any one of claims 1 to 9, wherein the biological sample is a sample of a subject comprising a tissue sample or a body liquid sample, such as a sample of a group of cells from a tumor, a tumor tissue, an RNA sample, a DNA sample, a blood sample, a serum sample, a plasma sample, a urine sample, a lymph fluid sample, a pleural fluid sample, or a brain liquor sample, preferably a sample of a group of cells from a tumor or a tumor tissue.

11. The use according to any one of claims 4 to 10, wherein the level, mutation status, and/or activity of said at least one m6A-mRNA modification biomarker and said at least one mcm5s2U-tRNA anticodon modification biomarker is determined by means of a detection method comprising mRNA analysis, DNA analysis, protein analysis, and/or a method for determining a single nucleotide polymorphism (SNP) in a target nucleic acid sequence, preferably wherein said method is selected from a method for determining a single nucleotide polymorphism (SNP) in a target nucleic acid sequence, such as a polymorphic array comprising a set of single nucleotide polymorphisms (SNPs), for example, an Affymetrix GeneChip, polymerase chain reaction (PCR), real-time quantitative PCR (qPCR), nanoString nCounter, enzyme-linked immunosorbent assay (ELISA), mass spectrometry, mass spectrometry immunoassay (MSIA), antibody-based protein chips, 2-dimensional gel electrophoresis, stable isotope standard capture with anti-peptide antibodies (SISCAPA), high-performance liquid chromatography (HPLC), genetic testing, western blot, cytometry bead array (CBA), radio immunoassay, and immunohistochemical staining, preferably wherein said detection method is a polymorphic array for determining a single nucleotide polymorphism (SNP) in a target nucleic acid sequence, and/or real-time quantitative PCR (qPCR).

12. The use according to any one of claims 6 to 11, wherein if the determined differential level, mutation status, and/or activity of said m6A-mRNA modification biomarker and/or mcm5s2U-tRNA anticodon modification biomarker in the biological sample from said subject is (i) such that the result is indicative for a lower level of m6A-modified mRNA and a higher level of mcm5s2U-modified tRNA, the time of progression free survival (PFS) and/or overall survival (OS) is reduced compared to a subject not showing such result, and/or (ii) such that the result is indicative for a higher level of m6A-modified mRNA and a lower level of mcm5s2U-modified tRNA, the time of progression free survival (PFS) and/or overall survival (OS) is prolonged compared to a subject not showing such result.

13. A use of a kit in a method for the prognosis, diagnosis, and/or monitoring of a disease, condition or disorder in a subject according to any one of the preceding claims, the kit comprising means for quantifying the level, mutation status, and/or activity of the at least one m6A-mRNA modification biomarker indicative and/or selective for the status and/or activity of the pathway regulating m6A-mRNA modification in a cell, and/or for quantifying the level, mutation status, and/or activity of the at least one mcm5s2U-tRNA anticodon modification biomarker indicative and/or selective for the status and/or activity of the pathway regulating mcm5s2U-tRNA anticodon modification in a cell.

14. The use according to claim 13, wherein (i) said at least one m6A-mRNA modification biomarker is selected from a gene, mRNA or protein of the m6A-MTC, an m6A reader protein, and an m6A eraser protein, and preferably is METTL3, METTL14, WTAP, VIRMA, RBM15, ZC3H13, YTHDF1, YTHDF2, YTHDF3, FTO, KIAA1429, CBLL1, RBM15, and/or ALKBH5s; and/or (ii) wherein said at least one mcm5s2U-tRNA anticodon modification biomarker is selected from a gene, mRNA or protein of ELP1/IKBKAP, ELP2, ELP3, ELP4, ELP5, ELP6, CTU1, CTU2, MOCS3, URM1, and/or ALKBH8.

## Patentansprüche

1. Verwendung eines Biomarkers, bestehend aus einer N6-Methyladenosin (m6A)-mRNA-Modifikation und einer 5-Methoxycarbonylmethyl-2-thiouridin (mcm5s2U)-tRNA-Antikodon-Modifikation in einem *in-vitro*-Verfahren für die Prognose, die Diagnose, die Stratifizierung und/oder die Überwachung einer Krankheit, eines Zustands oder einer Störung bei einem Subjekt.

2. Verwendung nach Anspruch 1, wobei das Verfahren ein Quantifizieren der Menge der m6A-mRNA-Modifikation und der mcm5s2U-tRNA-Antikodon-Modifikation direkt über ein Bewerten des modifizierten RNA-Nukleotids umfasst.

3. Verwendung nach Anspruch 2, wobei das Verfahren das Quantifizieren der Menge der m6A-mRNA-Modifikation und der mcm5s2U-tRNA-Antikodon-Modifikation direkt über das Bewerten des modifizierten RNA-Nukleotids mittels Massenspektrometrie umfasst.

4. Verwendung nach einem der Ansprüche 1 bis 3, wobei das Verfahren die Bestimmung des Niveaus von mindestens einem m6A-mRNA-Modifikations-Biomarker und des Niveaus, des Mutationsstatus und/oder der Aktivität von mindestens einem mcm5s2U-tRNA-Antikodon-Modifikations-Biomarker als einen kombinierten Biomarker umfasst, wobei der m6A-mRNA-Modifikations-Biomarker METTL3, METTL14, WTAP, VIRMA, RBM15, ZC3H13, YTHDF1, YTHDF2, YTHDF3, FTO, KIAA1429, CBLL1, RBM15 und/oder ALKBH5 ist; und der mcm5s2U-tRNA-Antikodon-Modifikations-Biomarker aus ELP1/IKBKAP, ELP2, ELP3, ELP4, ELP5, ELP6, CTU1, CTU2, MOCS3, URM1 und/oder ALKBH8 ausgewählt ist.

5. Verwendung nach Anspruch 4, wobei der mindestens eine mcm5s2U-tRNA-Antikodon-Modifikations-Biomarker ein Panel von CTU1, CTU2, MOCS3 und URM1 umfasst und/oder wobei der mindestens eine mcm5s2U-tRNA-Antikodon-Modifikations-Biomarker aus einem Panel von CTU1, CTU2, MOCS3 und URM1 besteht.

6. Verwendung nach einem der Ansprüche 1 bis 5, wobei das Verfahren für die Prognose, die Diagnose und/oder die Überwachung einer Krankheit, eines Zustands oder einer Störung bei einem Subjekt dient, umfassend die Schritte
(a) Bereitstellen einer erhaltenen biologischen Probe des Subjekts,
(b) Bestimmen des Niveaus, des Mutationsstatus und/oder der Aktivität von mindestens einem m6A-mRNA-Modifikations-Biomarker, der indikativ und/oder selektiv für den Status und/oder die Aktivität des Signalwegs ist, der die m6A-mRNA-Modifikation in einer Zelle in der biologischen Probe reguliert, durch Quantifizieren der Menge der m6A-Modifikation direkt über das Bewerten des modifizierten RNA-Nukleotids und/oder durch Quantifizieren des Vorhandenseins von mindestens einem m6A-mRNA-Modifikations-Biomarker, der aus METTL3, METTL14, WTAP, VIRMA, RBM15, ZC3H13, YTHDF1, YTHDF2, YTHDF3, FTO, KIAA1429, CBLL1, RBM15 und/oder ALKBH5 ausgewählt ist,
(c) Bestimmen des Niveaus, des Mutationsstatus und/oder der Aktivität von mindestens einem mcm5s2U-tRNA-Antikodon-Modifikations-Biomarker, der indikativ und/oder selektiv für den Status und/oder die Aktivität des Signalwegs ist, der die mcm5s2U-tRNA-Antikodon-Modifikation in einer Zelle in der biologischen Probe reguliert, durch Quantifizieren der Menge der mcm5s2U-tRNA-Antikodon-Modifikation direkt über das Bewerten des modifizierten RNA-Nukleotids und/oder durch Quantifizieren des Vorhandenseins von mindestens einem mcm5s2U-tRNA-Antikodon-Modifikations-Biomarker, der aus ELP1/IKBKAP, ELP2, ELP3, ELP4, ELP5, ELP6, CTU1, CTU2, MOCS3, URM1 und/oder ALKBH8 ausgewählt ist,
(d) Vergleichen des bestimmten Niveaus, des bestimmten Mutationsstatus und/oder der bestimmten Aktivität des m6A-mRNA-Modifikations-Biomarkers und des mcm5s2U-tRNA-Antikodon-Modifikations-Biomarkers in Schritten **(b)** und **(c)** mit einer Referenzprobe oder einem Referenzwert,
wobei ein differenzielles Niveau, ein differenzieller Mutationsstatus und/oder eine differenzielle Aktivität des m6A-mRNA-Modifikations-Biomarkers und/oder des mcm5s2U-tRNA-Antikodon-Modifikations-Biomarkers in der biologischen Probe von dem Subjekt im Vergleich zu der Referenzprobe oder dem Referenzwert für eine verkürzte Zeit eines progressionsfreien Überlebens (PFS) und/oder eines Gesamtüberlebens (OS) eines Patienten indikativ ist, der an einer Krankheit, einem Zustand oder einer Störung leidet, oder wobei ein differenzielles Niveau, ein differenzieller Mutationsstatus und/oder eine differenzielle Aktivität des m6A-mRNA-Modifikations-Biomarkers und/oder des mcm5s2U-tRNA-Antikodon-Modifikations-Biomarkers in der biologischen Probe von dem Subjekt im Vergleich zu der Referenzprobe oder dem Referenzwert für das Vorhandensein der Krankheit, des Zustands oder der Störung bei dem Subjekt indikativ ist.

7. Verwendung nach einem der Ansprüche 1 bis 5, wobei das Verfahren zum Stratifizieren eines Patienten für eine Behandlung dient, das Verfahren umfassend, als einen ersten Schritt, ein Identifizieren eines differenziellen Niveaus, eines differenziellen Mutationsstatus und/oder einer differenziellen Aktivität von mindestens einem m6A-mRNA-Modifikations-Biomarker und/oder mindestens einem mcm5s2U-tRNA-Antikodon-Modifikations-Biomarker in der biologischen Probe von dem Patienten im Vergleich zu einer Referenzprobe oder einem Referenzwert und, als einen zweiten Schritt, ein Entscheiden für oder gegen die Behandlung basierend auf dem differenziellen Niveau, dem differenziellen Mutationsstatus und/oder der differenziellen Aktivität, wobei das differenzielle Niveau, der differenzielle Mutationsstatus und/oder die differenzielle Aktivität durch Verwenden eines Verfahrens identifiziert wird, umfassend die Schritte:
(a) Bereitstellen einer erhaltenen biologischen Probe des Patienten,
(b) Bestimmen des Niveaus, des Mutationsstatus und/oder der Aktivität von mindestens einem m6A-mRNA-Modifikations-Biomarker, der indikativ und selektiv für den Status und/oder die Aktivität des Signalwegs ist, der die m6A-mRNA-Modifikation in einer Zelle in der biologischen Probe reguliert, durch Quantifizieren der Menge der m6A-Modifikation direkt über das Bewerten des modifizierten RNA-Nukleotids und/oder durch Quantifizieren des Vorhandenseins von mindestens einem m6A-mRNA-Modifikations-Biomarker, der aus METTL3, METTL14, WTAP, VIRMA, RBM15, ZC3H13, YTHDF1, YTHDF2, YTHDF3, FTO, KIAA1429, CBLL1, RBM15 und/oder ALKBH5 ausgewählt ist,
(c) Bestimmen des Niveaus, des Mutationsstatus und/oder der Aktivität von mindestens einem mcm5s2U-tRNA-Antikodon-Modifikations-Biomarker, der indikativ und selektiv für den Status und/oder die Aktivität des Signalwegs ist, der die mcm5s2U-tRNA-Antikodon-Modifikation in einer Zelle in der biologischen Probe reguliert, durch Quantifizieren der Menge der mcm5s2U-tRNA-Antikodon-Modifikation direkt über das Bewerten des modifizierten RNA-Nukleotids und/oder durch Quantifizieren des Vorhandenseins von mindestens einem mcm5s2U-tRNA-Antikodon-Modifikations-Biomarker, der aus ELP1/IKBKAP, ELP2, ELP3, ELP4, ELP5, ELP6, CTU1, CTU2, MOCS3, URM1 und/oder ALKBH8 ausgewählt ist, und
(d) Vergleichen des bestimmten Niveaus, des bestimmen Mutationsstatus und/oder der bestimmten Aktivität des m6A-mRNA-Modifikations-Biomarkers und des mcm5s2U-tRNA-Antikodon-Modifikations-Biomarkers in Schritten **(b)** und **(c)** mit einer Referenzprobe oder einem Referenzwert,
vorzugsweise, wobei die Behandlung eine Behandlung ist, die auf eine m6A-mRNA-Modifikation in einer Zelle und/oder eine mcm5s2U-tRNA-Antikodon-Modifikation in einer Zelle abzielt, vorzugsweise wobei die Behandlung das m6A-MTC, ein m6A-Leseprotein, ein m6A-Löschprotein und/oder den Elongatorkomplex hemmt oder aktiviert.

8. Verwendung nach Anspruch 7, wobei basierend auf dem differenziellen Niveau, dem differenziellen Mutationsstatus und/oder der differenziellen Aktivität des m6A-mRNA-Modifikations-Biomarkers und/oder des mcm5s2U-tRNA-Antikodon-Modifikations-Biomarkers in der biologischen Probe von dem Subjekt im Vergleich zu der Referenzprobe oder dem Referenzwert in Schritt **(d),** sich dafür entschieden wird, das Niveau, den Mutationsstatus und/oder die Aktivität des Signalwegs, der die m6A-mRNA-Modifikation reguliert, zu verstärken oder zu hemmen und/oder das Niveau, den Mutationsstatus und/oder die Aktivität des Signalwegs, der die mcm5s2U-tRNA-Antikodon-Modifikation reguliert, zu verstärken oder zu hemmen.

9. Verwendung nach einem der Ansprüche 1 bis 8, wobei die Krankheit, der Zustand oder die Störung Krebs ist und/oder eine Krankheit, die ein differenzielles Niveau, einen differenziellen Mutationsstatus und/oder eine differenzielle Aktivität des Signalwegs zeigt, der die m6A-mRNA-Modifikation in einer Zelle reguliert, und/oder die ein differenzielles Niveau, einen differenziellen Mutationsstatus und/oder eine differenzielle Aktivität des Signalwegs zeigt, der die mcm5s2U-tRNA-Antikodon-Modifikation in einer Zelle reguliert, vorzugsweise wobei der Krebs aus Brustkrebs, Melanom, Glioblastom, Gebärmutterschleimhautkrebs, akuter myeloischer Leukämie (AML), Lungenkrebs, Blasenkrebs, Leberzellkrebs, Medulloblastom, Nierenkrebs, Leberkrebs, Kopf- und Halskrebs, Prostatakrebs, Bauchspeicheldrüsenkrebs, Magenkrebs, Dickdarmkrebs, Schilddrüsenkrebs, Speiseröhrenkrebs, Hirnkrebs, Dickdarmkrebs, gastrischem Krebs, Gebärmutterhalskrebs, Eierstockkrebs, Harnwegskrebs, Nierenzellkrebs, Karzinom und einem Tumor ausgewählt ist, der ein differenzielles Niveau, einen differenziellen Mutationsstatus und/oder eine differenzielle Aktivität des Signalwegs zeigt, der die m6A-mRNA-Modifikation in einer Zelle reguliert und/oder die mcm5s2U-tRNA-Antikodon-Modifikation in einer Zelle, reguliert, mehr bevorzugt wobei der Krebs Brustkrebs ist.

10. Verwendung nach einem der Ansprüche 1 bis 9, wobei die biologische Probe eine Probe eines Subjekts ist, umfassend eine Gewebeprobe oder eine Körperflüssigkeitsprobe, wie eine Probe einer Zellgruppe aus einem Tumor, ein Tumorgewebe, eine RNA-Probe, eine DNA-Probe, eine Blutprobe, eine Serumprobe, eine Plasmaprobe, eine Urinprobe, eine Lymphfluidprobe, eine Pleurafluidprobe oder eine Hirnflüssigkeitsprobe, vorzugsweise eine Probe einer Zellgruppe aus einem Tumor oder einem Tumorgewebe.

11. Verwendung nach einem der Ansprüche 4 bis 10, wobei das Niveau, der Mutationsstatus und/oder die Aktivität des mindestens einen m6A-mRNA-Modifikations-Biomarkers und des mindestens einen mcm5s2U-tRNA-Antikodon-Modifikations-Biomarkers mittels eines Nachweisverfahrens bestimmt wird, umfassend eine mRNA-Analyse, eine DNA-Analyse, eine Proteinanalyse und/oder ein Verfahren zum Bestimmen eines Einzelnukleotidpolymorphismus (SNP) in einer Zielnukleinsäuresequenz, vorzugsweise wobei das Verfahren aus einem Verfahren zum Bestimmen eines Einzelnukleotidpolymorphismus (SNP) in einer Zielnukleinsäuresequenz, wie einem polymorphen Array, ausgewählt ist, umfassend einen Satz von Einzelnukleotidpolymorphismen (SNPs), zum Beispiel ein Affymetrix-GeneChip, Polymerasekettenreaktion (PCR), quantitative Echtzeit-PCR (qPCR), nanoString nCounter, Enzyme-verknüpftes immunoadsorbierendes Assay (ELISA), Massenspektrometrie, Massenspektrometrieimmunoassay (MSIA), antikörperbasierte Proteinchips, 2-dimensionale Gelelektrophorese, stabile Isotopenstandarderfassung mit Anti-Peptid-Antikörpern (SISCAPA), Hochleistungsflüssigchromatographie (HPLC), genetische Tests, Western Blot, Cytometrie-Bead-Array (CBA), Radioimmunoassay und immunhistochemische Färbung, vorzugsweise wobei das Nachweisverfahren ein polymorphes Array zum Bestimmen eines Einzelnukleotidpolymorphismus (SNP) in einer Zielnukleinsäuresequenz, und/oder quantitative Echtzeit-PCR (qPCR) ist.

12. Verwendung nach einem der Ansprüche 6 bis 11, wobei, falls das bestimmte differenzielle Niveau, der differenzielle Mutationsstatus und/oder die differenzielle Aktivität des m6A-mRNA-Modifikations-Biomarkers und/oder des mcm5s2U-tRNA-Antikodon-Modifikations-Biomarkers in der biologischen Probe von dem Subjekt (i) derart ist, dass das Ergebnis für ein niedrigeres Niveau von m6A-modifizierter mRNA und eines höheres Niveau von mcm5s2U-modifizierter tRNA indikativ ist, die Zeit des progressionsfreien Überlebens (PFS) und/oder des Gesamtüberlebens (OS) im Vergleich zu einem Subjekt verkürzt ist, das ein derartiges Ergebnis nicht zeigt, und/oder (ii) derart ist, dass das Ergebnis für ein höheres Niveau an m6A-modifizierter mRNA und ein niedrigeres Niveau von mcm5s2U-modifizierter tRNA indikativ ist, die Zeit des progressionsfreien Überlebens (PFS) und/oder des Gesamtüberlebens (OS) im Vergleich zu einem Subjekt verlängert ist, das ein derartiges Ergebnis nicht zeigt.

13. Verwendung eines Kits in einem Verfahren für die Prognose, die Diagnose und/oder die Überwachung einer Krankheit, eines Zustands oder einer Störung bei einem Subjekt nach einem der vorstehenden Ansprüche, das Kit umfassend Mittel zum Quantifizieren des Niveaus, des Mutationsstatus und/oder der Aktivität des mindestens einen m6A-mRNA-Modifikations-Biomarkers, der indikativ und/oder selektiv für den Status und/oder die Aktivität des Signalwegs ist, der die m6A-mRNA-Modifikation in einer Zelle reguliert, und/oder zum Quantifizieren des Niveaus, des Mutationsstatus und/oder der Aktivität des mindestens einen mcm5s2U-tRNA-Antikodon-Modifikations-Biomarkers, der indikativ und/oder selektiv für den Status und/oder die Aktivität des Signalwegs ist, der die mcm5s2U-tRNA-Antikodon-Modifikation in einer Zelle reguliert.

14. Verwendung nach Anspruch 13, wobei (i) der mindestens eine m6A-mRNA-Modifikations-Biomarker aus einem Gen, einer mRNA oder einem Protein des m6A-MTC, einem m6A-Leseprotein und einem m6A-Löschprotein ausgewählt ist und vorzugsweise METTL3, METTL14, WTAP, VIRMA, RBM15, ZC3H13, YTHDF1, YTHDF2, YTHDF3, FTO, KIAA1429, CBLL1, RBM15 und/oder ALKBH5s ist; und/oder (ii) wobei der mindestens eine mcm5s2U-tRNA-Antikodon-Modifikations-Biomarker aus einem Gen, einer mRNA oder einem Protein von ELP1/IKBKAP, ELP2, ELP3, ELP4, ELP5, ELP6, CTU1, CTU2, MOCS3, URM1 und/oder ALKBH8 ausgewählt ist.

## Revendications

1. Utilisation d'un biomarqueur constitué d'une modification d'ARNm par N6-méthyladénosine (m6A) et d'une modification d'anticodon d'ARNt par 5-méthoxycarbonylméthyl-2-thio-uridine (mcm5s2U) dans un procédé *in vitro* de pronostic, de diagnostic, de stratification et/ou de surveillance d'une maladie, d'un état ou d'un trouble chez un sujet.

2. Utilisation selon la revendication 1, dans laquelle le procédé comprend la quantification de la quantité de la modification d'ARNm par m6A et de la modification d'anticodon d'ARNt par m5s2U directement par l'évaluation du nucléotide d'ARN modifié.

3. Utilisation selon la revendication 2, dans laquelle le procédé comprend la quantification de la quantité de la modification d'ARNm par m6A et de la modification d'anticodon d'ARNt par m5s2U directement par l'évaluation du nucléotide d'ARN modifié par spectrométrie de masse.

4. Utilisation selon l'une quelconque des revendications 1 à 3, dans laquelle le procédé comprend la détermination du niveau d'au moins un biomarqueur de modification d'ARNm par m6A et du niveau, de l'état de mutation, et/ou de l'activité d'au moins un biomarqueur de modification d'anticodon d'ARNt par m5s2U comme biomarqueur combiné, dans laquelle le biomarqueur de modification d'ARNm par mc6A est METTL3, METTL14, WTAP, VIRMA, RBM15, ZC3H13, YTHDF1, YTHDF2, YTHDF3, FTO, KIAA1429, CBLL1, RBM15, et/ou ALKBH5 ; et le biomarqueur de modification d'anticodon d'ARNt par m5s2U est choisi parmi ELP1/IKBKAP, ELP2, ELP3, ELP4, ELP5, ELP6, CTU1, CTU2, MOCS3, URM1, et/ou ALKBH8.

5. Utilisation selon la revendication 4, dans laquelle ledit au moins un biomarqueur de modification d'anticodon d'ARN par mc5s2U comprend un panel de CTU1, CTU2, MOCS3, et URM1, et/ou dans laquelle ledit au moins un biomarqueur de modification d'anticodon d'ARN par mc5s2U consiste en un panel de CTU1, CTU2, MOCS3, et URM1.

6. Utilisation selon l'une quelconque des revendications 1 à 5, dans laquelle le procédé est destiné au pronostic, au diagnostic, et/ou au suivi d'une maladie, d'un état ou d'un trouble chez un sujet, comprenant les étapes consistant à
(a) Fournir un échantillon biologique obtenu dudit sujet,
(b) Déterminer le niveau, l'état de mutation, et/ou l'activité d'au moins un biomarqueur de modification d'ARNm par m6A indiquant et/ou étant sélectif de l'état et/ou de l'activité de la voie de régulation de modification d'ARNm par m6A dans une cellule dudit échantillon biologique, en quantifiant la quantité de la modification par m6A directement par l'évaluation du nucléotide d'ARN modifié et/ou en quantifiant la présence d'au moins un biomarqueur de modification d'ARNm par m6A choisi parmi METTL3, METTL14, WTAP, VIRMA, RBM15, ZC3H13, YTHDF1, YTHDF2, YTHDF3, FTO, KIAA1429, CBLL1, RBM15, et/ou ALKBH5,
(c) Déterminer le niveau, l'état de mutation, et/ou l'activité d'au moins un biomarqueur de modification d'anticodon d'ARNt par mc5s2U indiquant et/ou étant sélectif de l'état et/ou de l'activité de la voie de régulation de modification d'anticodon d'ARNt par mc5s2U dans une cellule dans ledit échantillon biologique, en quantifiant la quantité de la modification d'anticodon d'ARNt par m5s2U directement par l'évaluation du nucléotide d'ARN modifié et/ou en quantifiant la présence d'au moins un biomarqueur de modification d'anticodon d'ARNt par m5s2U choisi parmi ELP1/IKBKAP, ELP2, ELP3, ELP4, ELP5, ELP6, CTU1, CTU2, MOCS3, URM1, et/ou ALKBH8,
(d) Comparer le niveau, l'état de mutation, et/ou l'activité déterminé dudit biomarqueur de modification d'ARNm par m6A et dudit biomarqueur de modification d'anticodon d'ARNt par m5s2U aux étapes (b) et (c) avec un échantillon de référence ou une valeur de référence,
dans laquelle un niveau, un état de mutation, et/ou une activité différentiel dudit biomarqueur de modification d'ARNm par m6A et/ou dudit biomarqueur de modification d'anticodon d'ARNt par m5s2U dans l'échantillon biologique dudit sujet par rapport audit échantillon de référence ou à ladite valeur de référence indique une réduction de la durée de survie sans progression (SSP) et/ou de la survie globale (OS) d'un patient souffrant d'une maladie, d'un état ou d'un trouble, ou dans laquelle un niveau, un statut de mutation, et/ou une activité différentiel dudit biomarqueur de modification d'ARNm par m6A et/ou dudit biomarqueur de modification d'anticodon d'ARNt par m5s2U dans l'échantillon biologique dudit sujet par rapport audit échantillon de référence ou à ladite valeur de référence indique la présence de ladite maladie, dudit état ou dudit trouble chez ledit sujet.

7. Utilisation selon l'une quelconque des revendications 1 à 5, dans laquelle le procédé est destiné à stratifier un patient pour un traitement, ledit procédé comprenant, comme première étape, l'identification d'un niveau, d'un statut de mutation, et/ou d'une activité différentiel d'au moins un biomarqueur de modification d'ARNm par m6A et/ou d'au moins un biomarqueur de modification d'anticodon d'ARNt par m5s2U dans l'échantillon biologique dudit patient par rapport à un échantillon de référence ou à une valeur de référence, et, comme seconde étape, la décision en faveur ou contre ledit traitement sur la base dudit niveau, dudit statut de mutation, et/ou de ladite activité différentiel, dans laquelle ledit niveau, ledit statut de mutation, et/ou ladite activité différentiel est identifié en utilisant un procédé comprenant les étapes consistant à :
(a) Fournir un échantillon biologique obtenu dudit patient,
(b) Déterminer le niveau, l'état de mutation, et/ou l'activité d'au moins un biomarqueur de modification d'ARNm par m6A indiquant et étant sélectif de l'état et/ou de l'activité de la voie de régulation de modification d'ARNm par m6A dans une cellule dudit échantillon biologique, en quantifiant la quantité de la modification par m6A directement par l'évaluation du nucléotide d'ARN modifié et/ou en quantifiant la présence d'au moins un biomarqueur de modification d'ARNm par m6A choisi parmi METTL3, METTL14, WTAP, VIRMA, RBM15, ZC3H13, YTHDF1, YTHDF2, YTHDF3, FTO, KIAA1429, CBLL1, RBM15, et/ou ALKBH5,
(c) Déterminer le niveau, l'état de mutation, et/ou l'activité d'au moins un biomarqueur de modification d'anticodon d'ARNt par mc5s2U indiquant et étant sélectif de l'état et/ou de l'activité de la voie de régulation de modification d'anticodon d'ARNt par mc5s2U dans une cellule dans ledit échantillon biologique, en quantifiant la quantité de la modification d'anticodon d'ARNt par m5s2U directement par l'évaluation du nucléotide d'ARN modifié et/ou en quantifiant la présence d'au moins un biomarqueur de modification d'anticodon d'ARNt par m5s2U choisi parmi ELP1/IKBKAP, ELP2, ELP3, ELP4, ELP5, ELP6, CTU1, CTU2, MOCS3, URM1, et/ou ALKBH8, et
(d) Comparer le niveau, l'état de mutation, et/ou l'activité déterminé dudit biomarqueur de modification d'ARNm par m6A et dudit biomarqueur de modification d'anticodon d'ARNt par m5s2U aux étapes (b) et (c) avec un échantillon de référence ou une valeur de référence ;
de préférence, dans laquelle ledit traitement est un traitement ciblant une modification d'ARNm par m6A dans une cellule et/ou une modification d'anticodon d'ARNt par m5s2U dans une cellule, de préférence dans laquelle ledit traitement inhibe ou active le m6A-MTC, une protéine de lecture de m6A, une protéine d'effacement de m6A, et/ou le complexe d'élongation.

8. Utilisation selon la revendication 7, dans laquelle, sur la base du niveau, de l'état de mutation, et/ou de l'activité différentiel dudit biomarqueur de modification d'ARNm par m6A et/ou dudit biomarqueur de modification d'anticodon d'ARNt par m5s2U dans l'échantillon biologique dudit sujet par rapport audit échantillon de référence ou à ladite valeur à l'étape (d), il est décidé en faveur de l'amélioration ou de l'inhibition du niveau, de l'état de mutation, et/ou de l'activité de la voie de régulation de modification d'ARNm par m6A, et/ou de l'amélioration ou de l'inhibition du niveau, de l'état de mutation, et/ou de l'activité de la voie de régulation de modification d'anticodon d'ARNt par m5s2U.

9. Utilisation selon l'une quelconque des revendications 1 à 8, dans laquelle ladite maladie, ledit état ou ledit trouble est un cancer, et/ou une maladie qui présente un niveau, un statut de mutation, et/ou une activité différentiel de la voie de régulation de modification d'ARNm par m6A dans une cellule, et/ou qui présente un niveau, un statut de mutation, et/ou une activité différentiel de la voie de régulation de modification d'anticodon d'ARNt par m5s2U dans une cellule, de préférence dans laquelle ledit cancer est choisi parmi cancer du sein, mélanome, glioblastome, cancer de l'endomètre, leucémie myéloïde aiguë (LMA), cancer du poumon, cancer de la vessie, cancer hépatocellulaire, médulloblastome, cancer du rein, cancer du foie, cancer de la tête et du cou, cancer de la prostate, cancer du pancréas, cancer de l'estomac, cancer du côlon, cancer de la thyroïde, cancer de l'œsophage, cancer du cerveau, cancer colorectal, cancer gastrique, cancer du col de l'utérus, cancer de l'ovaire, cancer des voies urinaires, cancer du rein, carcinome, et une tumeur qui présente un niveau, un état de mutation, et/ou une activité différentiel de la voie de régulation de modification d'ARNm par m6A dans une cellule, et/ou de régulation de modification d'anticodon d'ARNt par m5s2U dans une cellule, plus préférablement dans laquelle ledit cancer est un cancer du sein.

10. Utilisation selon l'une quelconque des revendications 1 à 9, dans laquelle l'échantillon biologique est un échantillon d'un sujet comprenant un échantillon de tissu ou un échantillon de liquide corporel, tel qu'un échantillon d'un groupe de cellules provenant d'une tumeur, d'un tissu tumoral, d'un échantillon d'ARN, d'un échantillon d'ADN, d'un échantillon de sang, d'un échantillon de sérum, d'un échantillon de plasma, d'un échantillon d'urine, d'un échantillon de liquide lymphatique, d'un échantillon de liquide pleural, ou d'un échantillon de liqueur cérébrale, de préférence un échantillon d'un groupe de cellules provenant d'une tumeur ou d'un tissu tumoral.

11. Utilisation selon l'une quelconque des revendications 4 à 10, dans laquelle le niveau, l'état de mutation, et/ou l'activité dudit au moins un biomarqueur de modification d'ARNm par m6A et dudit au moins un biomarqueur de modification d'anticodon d'ARNt par m5s2U est déterminé au moyen d'un procédé de détection comprenant une analyse d'ARNm, une analyse d'ADN, une analyse de protéines, et/ou un procédé permettant de déterminer un polymorphisme mononucléotidique (SNP) dans une séquence d'acide nucléique cible, de préférence dans laquelle ledit procédé est choisi parmi un procédé de détermination d'un mononucléotidique (SNP) dans une séquence d'acide nucléique cible, telle qu'un réseau polymorphe comprenant un ensemble de polymorphismes mononucléotidiques (SNP), par exemple une puce GeneChip d'Affymetrix, une réaction en chaîne par polymérase (PCR), une PCR quantitative en temps réel (qPCR), un système nanoString nCounter, un essai immunoenzymatique (ELISA), une spectrométrie de masse, un essai immunologique par spectrométrie de masse (MSIA), des puces à protéines à base d'anticorps, une électrophorèse sur gel bidimensionnel, une capture d'étalons d'isotopes stables avec des anticorps anti-peptides (SISCAPA), une chromatographie liquide à haute performance (HPLC), des tests génétiques, un transfert Western, une cytométrie en réseau de billes (CBA), un essai radio-immunologique, et une coloration immunohistochimique, de préférence dans laquelle ledit procédé de détection est un réseau polymorphe permettant de déterminer un polymorphisme mononucléotidique (SNP) dans une séquence d'acide nucléique cible, et/ou une PCR quantitative en temps réel (qPCR).

12. Utilisation selon l'une quelconque des revendications 6 à 11, dans laquelle, si le niveau, l'état de mutation, et/ou l'activité différentiel déterminé dudit biomarqueur de modification d'ARNm par m6A et/ou dudit biomarqueur de modification d'anticodon d'ARNt par m5s2U dans l'échantillon biologique dudit sujet est (i) tel que le résultat indique un niveau inférieur d'ARNm modifié par m6A et un niveau supérieur d'ARNt modifié par mcm5s2U, la durée de survie sans progression (SSP) et/ou de la survie globale (OS) est réduite par rapport à un sujet ne présentant pas un tel résultat, et/ou (ii) tel que le résultat indique un niveau supérieur d'ARNm modifié par m6A et un niveau inférieur d'ARNt modifié par mcm5s2U, la durée de survie sans progression (SSP) et/ou de la survie globale (OS) est prolongée par rapport à un sujet ne présentant pas un tel résultat.

13. Utilisation d'une trousse dans un procédé de pronostic, de diagnostic, et/ou de suivi d'une maladie, d'un état ou d'un trouble chez un sujet selon l'une quelconque des revendications précédentes, la trousse comprenant des moyens permettant de quantifier le niveau, l'état de mutation, et/ou l'activité de l'au moins un biomarqueur de modification d'ARNm par m6A indiquant et/ou étant sélectif de l'état et/ou de l'activité de la voie de régulation de modification d'ARNm par m6A dans une cellule, et/ou permettant de quantifier le niveau, l'état de mutation, et/ou l'activité de l'au moins un biomarqueur de modification d'anticodon d'ARN par mc5s2U indiquant et/ou étant sélectif de l'état et/ou de l'activité de la voie de régulation de modification d'anticodon d'ARN par mc5s2U dans une cellule.

14. Utilisation selon la revendication 13, dans laquelle (i) ledit au moins un biomarqueur de modification d'ARNm par m6A est choisi parmi un gène, un ARNm ou une protéine du m6A-MTC, une protéine de lecture de m6A, et une protéine d'effacement de m6A, et est de préférence METTL3, METTL14, WTAP, VIRMA, RBM15, ZC3H13, YTHDF1, YTHDF2, YTHDF3, FTO, KIAA1429, CBLL1, RBM15, et/ou ALKBH5s ; et/ou (ii) dans laquelle ledit au moins un biomarqueur de modification d'anticodon d'ARNt par m5s2U est choisi parmi un gène, un ARNm ou une protéine de ELP1/IKBKAP, ELP2, ELP3, ELP4, ELP5, ELP6, CTU1, CTU2, MOCS3, URM1, et/ou ALKBH8.
